# EUROPEAN PATENT SPECIFICATION

(11) **EP 3 178 512 B1**
(45) Date of publication and mention of the grant of the patent: **28.08.2019**
(21) Application number: 15199382.1
(22) Date of filing: 10.12.2015
(51) Int. Cl.: A61M 16/06

(54) **ADAPTABLE PATIENT INTERFACE**
ANPASSBARE PATIENTENSCHNITTSTELLE
INTERFACE PATIENT ADAPTABLE

(43) Date of publication of application: 14.06.2017
(73) Proprietor: ResMed Pty Ltd, Bella Vista, New South Wales 2153 (AU)
(72) Inventor: Zeppenfeld, Matthias, 82515 Wolfratshausen (DE); Burz, Sebastian, 87656 Germaringen (DE); Lang, Bernd, 82166 Gräfelfing (DE); Bayer, Christian, 82377 Penzberg (DE)
(74) Representative: Vossius & Partner Patentanwälte Rechtsanwälte mbB

(56) References cited:
- WO-A2-2013/128324
- AU-A4- 2014 100 361
- CN-A- 103 536 996
- US-A1- 2012 216 813
- US-A1- 2013 133 664

## Description

### 1 BACKGROUND OF THE INVENTION

### 1.1 FIELD OF THE INVENTION

The invention relates to one or more of the detection, diagnosis, treatment, prevention and amelioration of respiratory-related disorders. The invention also relates to medical devices or apparatus, and their use. In particular, the invention relates to a patient interface for delivering a supply of breathable gas to a patient.

### 1.2 DESCRIPTION OF THE RELATED ART

### 1.2.1 Human Respiratory System and its Disorders

The respiratory system of the body facilitates gas exchange. The nose and mouth form the entrance to the airways of a patient.

The airways include a series of branching tubes, which become narrower, shorter and more numerous as they penetrate deeper into the lung. The prime function of the lung is gas exchange, allowing oxygen to move from the air into the venous blood and carbon dioxide to move out. The trachea divides into right and left main bronchi, which further divide eventually into terminal bronchioles. The bronchi make up the conducting airways, and do not take part in gas exchange. Further divisions of the airways lead to the respiratory bronchioles, and eventually to the alveoli. The alveolated region of the lung is where the gas exchange takes place, and is referred to as the respiratory zone. See *"*Respiratory Physiology", by John B. West, Lippincott Williams & Wilkins, 9th edition published 2011.

A range of respiratory disorders exist. Certain disorders may be characterised by particular events, e.g. apneas, hypopneas, and hyperpneas.

Obstructive Sleep Apnea (OSA), a form of Sleep Disordered Breathing (SDB), is characterized by events including occlusion or obstruction of the upper air passage during sleep. It results from a combination of an abnormally small upper airway and the normal loss of muscle tone in the region of the tongue, soft palate and posterior oropharyngeal wall during sleep. The condition causes the affected patient to stop breathing for periods typically of 30 to 120 seconds in duration, sometimes 200 to 300 times per night. It often causes excessive daytime somnolence, and it may cause cardiovascular disease and brain damage. The syndrome is a common disorder, particularly in middle aged overweight males, although a person affected may have no awareness of the problem. *See* US Patent No. 4,944,310 (Sullivan).

Cheyne-Stokes Respiration (CSR) is another form of sleep disordered breathing. CSR is a disorder of a patient's respiratory controller in which there are rhythmic alternating periods of waxing and waning ventilation known as CSR cycles. CSR is characterised by repetitive de-oxygenation and re-oxygenation of the arterial blood. It is possible that CSR is harmful because of the repetitive hypoxia. In some patients CSR is associated with repetitive arousal from sleep, which causes severe sleep disruption, increased sympathetic activity, and increased afterload. *See* US Patent No. 6,532,959 (Berthon-Jones).

Obesity Hyperventilation Syndrome (OHS) is defined as the combination of severe obesity and awake chronic hypercapnia, in the absence of other known causes for hypoventilation. Symptoms include dyspnea, morning headache and excessive daytime sleepiness.

Chronic Obstructive Pulmonary Disease (COPD) encompasses any of a group of lower airway diseases that have certain characteristics in common. These include increased resistance to air movement, extended expiratory phase of respiration, and loss of the normal elasticity of the lung. Examples of COPD are emphysema and chronic bronchitis. COPD is caused by chronic tobacco smoking (primary risk factor), occupational exposures, air pollution and genetic factors. Symptoms include: dyspnea on exertion, chronic cough and sputum production.

Neuromuscular Disease (NMD) is a broad term that encompasses many diseases and ailments that impair the functioning of the muscles either directly via intrinsic muscle pathology, or indirectly via nerve pathology. Some NMD patients are characterised by progressive muscular impairment leading to loss of ambulation, being wheelchair-bound, swallowing difficulties, respiratory muscle weakness and, eventually, death from respiratory failure. Neuromuscular disorders can be divided into rapidly progressive and slowly progressive: (i) Rapidly progressive disorders: Characterised by muscle impairment that worsens over months and results in death within a few years (e.g. Amyotrophic lateral sclerosis (ALS) and Duchenne muscular dystrophy (DMD) in teenagers); (ii) Variable or slowly progressive disorders: Characterised by muscle impairment that worsens over years and only mildly reduces life expectancy (e.g. Limb girdle, Facioscapulohumeral and Myotonic muscular dystrophy). Symptoms of respiratory failure in NMD include: increasing generalised weakness, dysphagia, dyspnea on exertion and at rest, fatigue, sleepiness, morning headache, and difficulties with concentration and mood changes.

Chest wall disorders are a group of thoracic deformities that result in inefficient coupling between the respiratory muscles and the thoracic cage. The disorders are usually characterised by a restrictive defect and share the potential of long term hypercapnic respiratory failure. Scoliosis and/or kyphoscoliosis may cause severe respiratory failure. Symptoms of respiratory failure include: dyspnea on exertion, peripheral oedema, orthopnea, repeated chest infections, morning headaches, fatigue, poor sleep quality and loss of appetite.

A range of therapies have been used to treat or ameliorate such conditions. Furthermore, otherwise healthy individuals may take advantage of such therapies to prevent respiratory disorders from arising. However, these have a number of shortcomings.

### 1.2.2 Therapy

Continuous Positive Airway Pressure (CPAP) therapy has been used to treat Obstructive Sleep Apnea (OSA). The hypothesis is that continuous positive airway pressure acts as a pneumatic splint and may prevent upper airway occlusion by pushing the soft palate and tongue forward and away from the posterior oropharyngeal wall. Treatment of OSA by CPAP therapy may be voluntary, and hence patients may elect not to comply with therapy if they find devices used to provide such therapy one or more of: uncomfortable, difficult to use, expensive and aesthetically unappealing.

Non-invasive ventilation (NIV) provides ventilatory support to a patient through the upper airways to assist the patient in taking a full breath and/or maintain adequate oxygen levels in the body by doing some or all of the work of breathing. The ventilatory support is provided via a patient interface. NIV has been used to treat CSR, OHS, COPD, MD and Chest Wall disorders. In some forms, the comfort and effectiveness of these therapies may be improved.

Invasive ventilation (IV) provides ventilatory support to patients that are no longer able to effectively breathe themselves and may be provided using a tracheostomy tube. In some forms, the comfort and effectiveness of these therapies may be improved.

### 1.2.3 Diagnosis and Treatment Systems

These therapies may be provided by a treatment system or device. Systems and devices may also be used to diagnose a condition without treating it.

A treatment system may comprise a Respiratory Pressure Therapy Device (RPT device), an air circuit, a humidifier, a patient interface, and data management.

Another form of treatment system is a mandibular repositioning device.

### 1.2.3.1 Patient Interface

A patient interface may be used to interface respiratory equipment to its wearer, for example by providing a flow of air to an entrance to the airways. The flow of air may be provided via a mask to the nose and/or mouth, a tube to the mouth or a tracheostomy tube to the trachea of a patient. Depending upon the therapy to be applied, the patient interface may form a seal, e.g., with a region of the patient's face, to facilitate the delivery of gas at a pressure at sufficient variance with ambient pressure to effect therapy, e.g., at a positive pressure of about 10 cmH₂O relative to ambient pressure. For other forms of therapy, such as the delivery of oxygen, the patient interface may not include a seal sufficient to facilitate delivery to the airways of a supply of gas at a positive pressure of about 10 cmH₂O.

The design of a patient interface presents a number of challenges. The face has a complex three-dimensional shape. The size and shape of noses varies considerably between individuals. Since the head includes bone, cartilage and soft tissue, different regions of the face respond differently to mechanical forces. The jaw or mandible may move relative to other bones of the skull. The whole head may move during the course of a period of respiratory therapy.

As a consequence of these challenges, some masks suffer from being one or more of obtrusive, aesthetically undesirable, costly, poorly fitting, difficult to use, and uncomfortable especially when worn for long periods of time or when a patient is unfamiliar with a system. For example, masks designed solely for aviators, masks designed as part of personal protection equipment (e.g. filter masks), SCUBA masks, or for the administration of anaesthetics may be tolerable for their original application, but nevertheless such masks may be undesirably uncomfortable to be worn for extended periods of time, e.g., several hours. This discomfort may lead to a reduction in patient compliance with therapy. This is even more so if the mask is to be worn during sleep.

CPAP therapy is highly effective to treat certain respiratory disorders, provided patients comply with therapy. If a mask is uncomfortable, or difficult to use a patient may not comply with therapy. Since it is often recommended that a patient regularly wash their mask, if a mask is difficult to clean (e.g., difficult to assemble or disassemble), patients may not clean their mask and this may impact on patient compliance.

While a mask for other applications (e.g. aviators) may not be suitable for use in treating sleep disordered breathing, a mask designed for use in treating sleep disordered breathing may be suitable for other applications.

For these reasons, patient interfaces for delivery of CPAP during sleep form a distinct field.

### 1.2.3.1.1 Seal-forming portion

Patient interfaces may include a seal-forming portion. Since it is in direct contact with the patient's face, the shape and configuration of the seal-forming portion can have a direct impact the effectiveness and comfort of the patient interface.

A patient interface may be partly characterised according to the design intent of where the seal-forming portion is to engage with the face in use. In one form of patient interface, a seal-forming portion may comprise two sub-portions to engage with respective left and right nares. In one form of patient interface, a seal-forming portion may comprise a single element that surrounds both nares in use. Such single element may be designed to for example overlay an upper lip region and a nasal bridge region of a face. In one form of patient interface a seal-forming portion may comprise an element that surrounds a mouth region in use, e.g. by forming a seal on a lower lip region of a face. In one form of patient interface, a seal-forming portion may comprise a single element that surrounds both nares and a mouth region in use. These different types of patient interfaces may be known by a variety of names by their manufacturer including nasal masks, full-face masks, nasal pillows, nasal puffs and oro-nasal masks.

A seal-forming portion that may be effective in one region of a patient's face may be inappropriate in another region, e.g. because of the different shape, structure, variability and sensitivity regions of the patient's face. For example, a seal on swimming goggles that overlays a patient's forehead may not be appropriate to use on a patient's nose.

Certain seal-forming portions may be designed for mass manufacture such that one design fit and be comfortable and effective for a wide range of different face shapes and sizes. To the extent to which there is a mismatch between the shape of the patient's face, and the seal-forming portion of the mass-manufactured patient interface, one or both must adapt in order for a seal to form.

One type of seal-forming portion extends around the periphery of the patient interface, and is intended to seal against the patient's face when force is applied to the patient interface with the seal-forming portion in confronting engagement with the patient's face. The seal-forming portion may include an air or fluid filled cushion, or a moulded or formed surface of a resilient seal element made of an elastomer such as a rubber. With this type of seal-forming portion, if the fit is not adequate, there will be gaps between the seal-forming portion and the face, and additional force will be required to force the patient interface against the face in order to achieve a seal.

Another type of seal-forming portion incorporates a flap seal of thin material positioned about the periphery of the mask so as to provide a self-sealing action against the face of the patient when positive pressure is applied within the mask. Like the previous style of seal forming portion, if the match between the face and the mask is not good, additional force may be required to achieve a seal, or the mask may leak. Furthermore, if the shape of the seal-forming portion does not match that of the patient, it may crease or buckle in use, giving rise to leaks.

Another type of seal-forming portion may comprise a friction-fit element, e.g. for insertion into a naris, however some patients find these uncomfortable.

Another form of seal-forming portion may use adhesive to achieve a seal. Some patients may find it inconvenient to constantly apply and remove an adhesive to their face.

A range of patient interface seal-forming portion technologies are disclosed in the following patent applications, assigned to ResMed Limited: WO 1998/004,310; WO 2006/074,513; WO 2010/135,785.

One form of nasal pillow is found in the Adam Circuit manufactured by Puritan Bennett. Another nasal pillow, or nasal puff is the subject of US Patent 4,782,832 (Trimble et al.)*,* assigned to Puritan-Bennett Corporation.

ResMed Limited has manufactured the following products that incorporate nasal pillows: SWIFT™ nasal pillows mask, SWIFT™ II nasal pillows mask, SWIFT™ LT nasal pillows mask, SWIFT™ FX nasal pillows mask and MIRAGE LIBERTY™ full-face mask. The following patent applications, assigned to ResMed Limited, describe examples of nasal pillows masks: International Patent Application WO2004/073,778 (describing amongst other things aspects of the ResMed Limited SWIFT™ nasal pillows), US Patent Application 2009/0044808 (describing amongst other things aspects of the ResMed Limited SWIFT™ LT nasal pillows); International Patent Applications WO 2005/063,328 and WO 2006/130,903 (describing amongst other things aspects of the ResMed Limited MIRAGE LIBERTY™ full-face mask); International Patent Application WO 2009/052,560 (describing amongst other things aspects of the ResMed Limited SWIFT™ FX nasal pillows).

### 1.2.3.1.2 Positioning and stabilising

A seal-forming portion of a patient interface used for positive air pressure therapy is subject to the corresponding force of the air pressure to disrupt a seal. Thus a variety of techniques have been used to position the seal-forming portion, and to maintain it in sealing relation with the appropriate portion of the face.

One technique is the use of adhesives. See for example US Patent Application Publication No. US 2010/0000534. However, the use of adhesives may be uncomfortable for some.

Another technique is the use of one or more straps and/or stabilising harnesses. Many such harnesses suffer from being one or more of ill-fitting, bulky, uncomfortable and awkward to use.

A further technique used to stabilise seal and also distribute the compressive force of a seal-forming portion of a patient interface is to utilise a forehead support. One problem that arises when sealing a mask to a patient's face is that when headgear straps are tightened to fit the mask it results in over compressions of the seal forming portions against the face in areas such as the wearer's nose. In addition, the mask may move around during use effecting seal on the face. Thus, to distribute the compressive force and increase stability, an additional point of facial contact is provided by way of a forehead support that rests against the patient's forehead.

Respiratory masks used in CPAP therapy are normally designed such that the mask fits to the largest proportion of the patient population taking into account facial variations. In particular, in the field of full face respiratory masks, which cover the mouth of the patient in addition to his/her nose, the distance in which the forehead protrudes from the plane of the face and the angle in which it protrudes is a crucial factor for positioning the forehead support to effectively support fit and seal. Among patients, this influencing factor varies most significantly; moreover, this variation cannot be solely compensated for by the elasticity of the sealing membrane of the mask.

It is known to provide an adjustable forehead support mechanism positioned at the top of the mask in close proximity to the patient's forehead. The forehead support generally is positioned against the forehead by straps. The patient interface is adjusted to the particular shape of the patient by extending or retracting the forehead support vertically in the plane of the face or perpendicular to the plane of the face to adjust for facial variations. The adjustment mechanisms are usually in multiple part assemblies, can be bulky, obtrusive and difficult to disassemble for cleaning/removal. Often the forehead support adjustment mechanism comprises a dial and locking mechanism leading to complex assemblies. More parts also lead to difficulty in handling and adjustment by the user. For instance, US patent number 6,532,961 discloses an adjustable forehead support.

### 1.2.3.1.3 Vent technologies

Some forms of patient interface systems may include a vent to allow the washout of exhaled carbon dioxide. The vent may allow a flow of gas from an interior space of the patient interface, e.g., the plenum chamber, to an exterior of the patient interface, e.g., to ambient. The vent may comprise an orifice and gas may flow through the orifice in use of the mask. Many such vents are noisy. Others may become blocked in use and thus provide insufficient washout. Some vents may be disruptive of the sleep of a bed-partner 1100 of the patient 1000, e.g. through noise or focussed airflow.

ResMed Limited has developed a number of improved mask vent technologies. *See* International Patent Application Publication No. WO 1998/034,665; International Patent Application Publication No. WO 2000/078,381; US Patent No. 6,581,594; US Patent Application Publication No. US 2009/0050156; US Patent Application Publication No. 2009/0044808.

**Table of noise of prior masks (ISO 17510-2:2007, 10 cmH₂O pressure at 1m)**

| Mask name | Mask type | A-weighted sound power level dB(A) (uncertainty) | A-weighted sound pressure dB(A) (uncertainty) | Year (approx.) |
|---|---|---|---|---|
| Glue-on (*) | nasal | 50.9 | 42.9 | 1981 |
| ResCare standard (*) | nasal | 31.5 | 23.5 | 1993 |
| ResMed Mirage™ (*) | nasal | 29.5 | 21.5 | 1998 |
| ResMed UltraMirage ™ | nasal | 36 (3) | 28 (3) | 2000 |
| ResMed Mirage Activa™ | nasal | 32 (3) | 24 (3) | 2002 |
| ResMed Mirage Micro™ | nasal | 30 (3) | 22 (3) | 2008 |
| ResMed Mirage ™ SoftGel | nasal | 29 (3) | | 2008 |
| ResMed Mirage™ FX | nasal | 26 (3) | 18 (3) | 2010 |
| ResMed Mirage Swift™ (*) | nasal pillows | 37 | 29 | 2004 |
| ResMed Mirage Swift™ II | nasal pillows | 28 (3) | 20 (3) | 2005 |
| ResMed Mirage Swift™ LT | nasal pillows | 25 (3) | 17 (3) | 2008 |
| ResMed AirFit P10 | nasal pillows | 21 (3) | 13 (3) | 2014 |

| | | | | |
|---|---|---|---|---|
| (* one specimen only, measured using test method specified in ISO3744 in CPAP mode at 10 cmH₂O)Sound pressure values of a variety of objects are listed below | | | | |

| Object | A-weighted sound pressure dB(A) | Notes |
|---|---|---|
| Vacuum cleaner: Nilfisk Walter Broadly Litter Hog: B+ Grade | 68 | ISO3744 at 1m distance |
| Conversational speech | 60 | 1m distance |
| home Average | 50 | |
| Quiet library | 40 | |
| Quiet bedroom at night | 30 | |
| Background in TV studio | 20 | |

### 1.2.3.2 Respiratory Pressure Therapy (RPT) Device

Air pressure generators are known in a range of applications, e.g. industrial-scale ventilation systems. However, air pressure generators for medical applications have particular requirements not fulfilled by more generalised air pressure generators, such as the reliability, size and weight requirements of medical devices. In addition, even devices designed for medical treatment may suffer from shortcomings, pertaining to one or more of: comfort, noise, ease of use, efficacy, size, weight, manufacturability, cost, and reliability.

An example of the special requirements of certain RPT devices is acoustic noise.

**Table of noise output levels of prior RPT devices (one specimen only, measured using test method specified in ISO3744 in CPAP mode at 10 cmH₂O).**

| RPT Device name | A-weighted sound power level dB(A) | Year (approx.) |
|---|---|---|
| C-Series Tango™ | 31.9 | 2007 |
| C-Series Tango™ with Humidifier | 33.1 | 2007 |
| S8 Escape™ II | 30.5 | 2005 |
| S8 Escape™ II with H4i™ Humidifier | 31.1 | 2005 |
| S9 AutoSet™ | 26.5 | 2010 |
| S9 AutoSet™ with H5i Humidifier | 28.6 | 2010 |

One known RPT device used for treating sleep disordered breathing is the S9 Sleep Therapy System, manufactured by ResMed Limited. Another example of an RPT device is a ventilator. Ventilators such as the ResMed Stellar™ Series of Adult and Paediatric Ventilators may provide support for invasive and non-invasive non-dependent ventilation for a range of patients for treating a number of conditions such as but not limited to NMD, OHS and COPD.

The ResMed Elisée™ 150 ventilator and ResMed VS III™ ventilator may provide support for invasive and non-invasive dependent ventilation suitable for adult or paediatric patients for treating a number of conditions. These ventilators provide volumetric and barometric ventilation modes with a single or double limb circuit. RPT devices typically comprise a pressure generator, such as a motor-driven blower or a compressed gas reservoir, and are configured to supply a flow of air to the airway of a patient. In some cases, the flow of air may be supplied to the airway of the patient at positive pressure. The outlet of the RPT device is connected via an air circuit to a patient interface such as those described above.

### 1.2.3.3 Humidifier

Delivery of a flow of air without humidification may cause drying of airways. The use of a humidifier with an RPT device and the patient interface produces humidified gas that minimizes drying of the nasal mucosa and increases patient airway comfort. In addition in cooler climates, warm air applied generally to the face area in and about the patient interface is more comfortable than cold air. A range of artificial humidification devices and systems are known, however they may not fulfil the specialised requirements of a medical humidifier.

Medical humidifiers are used to increase humidity and/or temperature of the flow of air in relation to ambient air when required, typically where the patient may be asleep or resting (e.g. at a hospital). A medical humidifier for bedside placement may be small. A medical humidifier may be configured to only humidify and/or heat the flow of air delivered to the patient without humidifying and/or heating the patient's surroundings. Room-based systems (e.g. a sauna, an air conditioner, or an evaporative cooler), for example, may also humidify air that is breathed in by the patient, however those systems would also humidify and/or heat the entire room, which may cause discomfort to the occupants. Furthermore medical humidifiers may have more stringent safety constraints than industrial humidifiers

While a number of medical humidifiers are known, they can suffer from one or more shortcomings. Some medical humidifiers may provide inadequate humidification, some are difficult or inconvenient to use by patients.

### 1.2.3.4 Mandibular repositioning

A mandibular repositioning device (MRD) or mandibular advancement device (MAD) is one of the treatment options for sleep apnea and snoring. It is an adjustable oral appliance available from a dentist or other supplier that holds the lower jaw (mandible) in a forward position during sleep. The MRD is a removable device that a patient inserts into their mouth prior to going to sleep and removes following sleep. Thus, the MRD is not designed to be worn all of the time. The MRD may be custom made or produced in a standard form and includes a bite impression portion designed to allow fitting to a patient's teeth. This mechanical protrusion of the lower jaw expands the space behind the tongue, puts tension on the pharyngeal walls to reduce collapse of the airway and diminishes palate vibration.

In certain examples a mandibular advancement device may comprise an upper splint that is intended to engage with or fit over teeth on the upper jaw or maxilla and a lower splint that is intended to engage with or fit over teeth on the upper jaw or mandible. The upper and lower splints are connected together laterally via a pair of connecting rods. The pair of connecting rods are fixed symmetrically on the upper splint and on the lower splint.

In such a design the length of the connecting rods is selected such that when the MRD is placed in a patient's mouth the mandible is held in an advanced position. The length of the connecting rods may be adjusted to change the level of protrusion of the mandible. A dentist may determine a level of protrusion for the mandible that will determine the length of the connecting rods.

Some MRDs are structured to push the mandible forward relative to the maxilla while other MADs, such as the ResMed Narval CC™ MRD are designed to retain the mandible in a forward position. This device also reduces or minimises dental and temporo-mandibular joint (TMJ) side effects. Thus, it is configured to minimises or prevent any movement of one or more of the teeth.

### 1.2.4 Monitoring Systems

Polysomnography (PSG) is a conventional system for diagnosis and prognosis of cardio-pulmonary disorders. PSG typically involves the placement of 15 to 20 contact sensors on a person in order to record various bodily signals such as electroencephalography (EEG), electrocardiography (ECG), electrooculograpy (EOG), etc. However, while they may be suitable for their usual application in a clinical setting, such systems are complicated and potentially expensive, and / or may be uncomfortable or impractical for a patient at home trying to sleep.

The designer of a device may be presented with an infinite number of choices to make to design a product or system. Design criteria often conflict, meaning that certain design choices are far from routine or inevitable. Furthermore, the comfort and efficacy of certain aspects may be highly sensitive to small, subtle changes in one or more parameters.

### 2 BRIEF SUMMARY OF THE INVENTION

The invention is directed towards providing medical devices used in the diagnosis, amelioration, treatment, or prevention of respiratory disorders having one or more of improved comfort, cost, efficacy, ease of use and manufacturability.

A first aspect of the invention relates to apparatus used in the diagnosis, amelioration, treatment or prevention of a respiratory disorder.

An aspect of certain forms of the invention is to provide apparatus that improve the compliance of patients with respiratory therapy.

Another aspect of one form of the invention is to provide a patient interface being adapted to fit to a large number of patient and being better adaptable to the individual anatomy of the patients head.

Another aspect of one form of the invention is to simplify the adjustable forehead support mechanism on a mask while maintaining its level of adjustability. Another aspect of one form of the invention is to reduce the manufacturing costs, e.g. by reducing the number of parts and allowing easier assembly. Yet another aspect is to improve the ease of use of the patient interface, e.g. by an easier (dis-)assembly, and to reduce the obtrusiveness of the device.

Another aspect of one form of the invention is a patient interface that is moulded or otherwise constructed with a clearly defined perimeter shape which is intended to match that of an intended wearer.

An aspect of one form of the invention is a patient interface that may be washed in a home of a patient, e.g., in soapy water, without requiring specialised cleaning equipment.

In particular, the invention relates to a patient interface.

One or more objects of the invention is/are solved by the subject-matter of the independent claim 1. Further embodiments, aspects and advantages of the invention are described in the dependent claims and the below description.

The invention may also be described by the following numbered aspects while the comments in between individual aspects are understood to contain further additional aspects:
1. A patient interface (3000) for delivering a supply of breathable gas to the patient's airways comprising:
   a seal-forming structure (3100) adapted to seal against a user's face;
   a shell (3250) being connected to the seal-forming structure (3100);
   a frame (3800) adapted to be connected with a headgear (3300); wherein the seal-forming structure (3100) and the shell (3250) form at least a part of a mask plenum chamber (3200);
      and
   wherein the frame (3800) is coupled to the shell (3250) so as to allow a relative movement of the frame (3800) with respect to the shell (3250) ;
   wherein the patient interface comprises means for providing the relative movement being configured
      - as a sliding arrangement (3500) and wherein the sliding arrangement (3500) is configured such that the frame (3800) slides relative to the shell (3250) along a curved path with a fulcrum (A'-A') or
      - as a joint (3900) adapted to rotate around a rotation axis (A-A); and
   wherein the fulcrum (A'-A') of the sliding arrangement (3500) or the rotation axis (A-A) of the joint (3900) is located in or adjacent the center of gravity of the shell (3250) with seal-forming structure (3100).

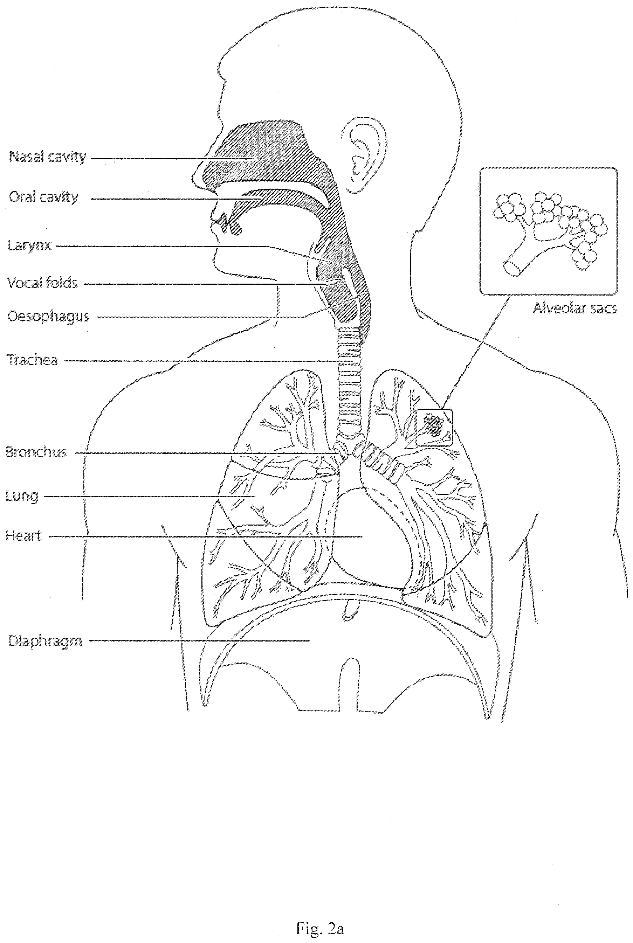

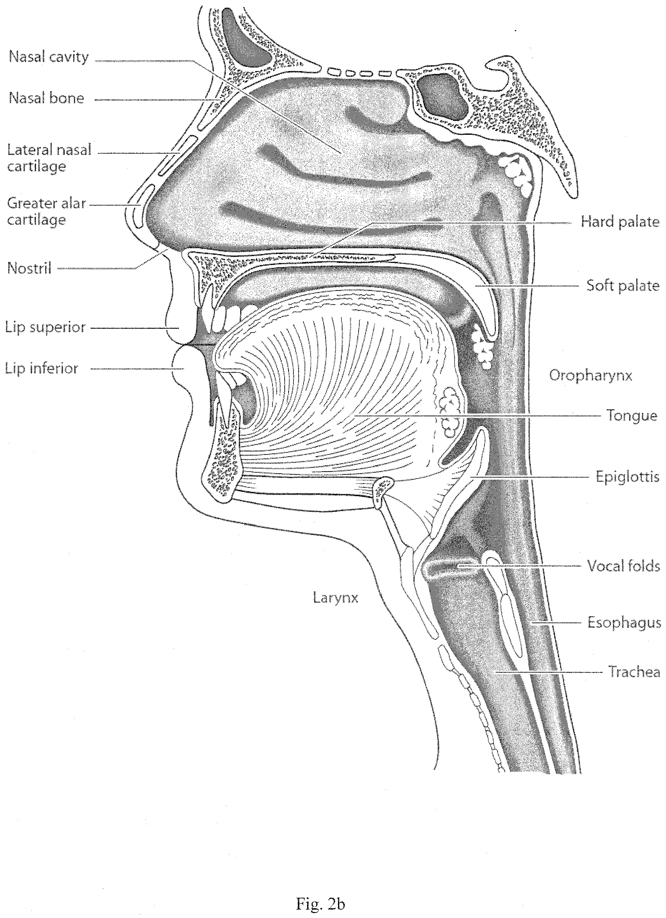

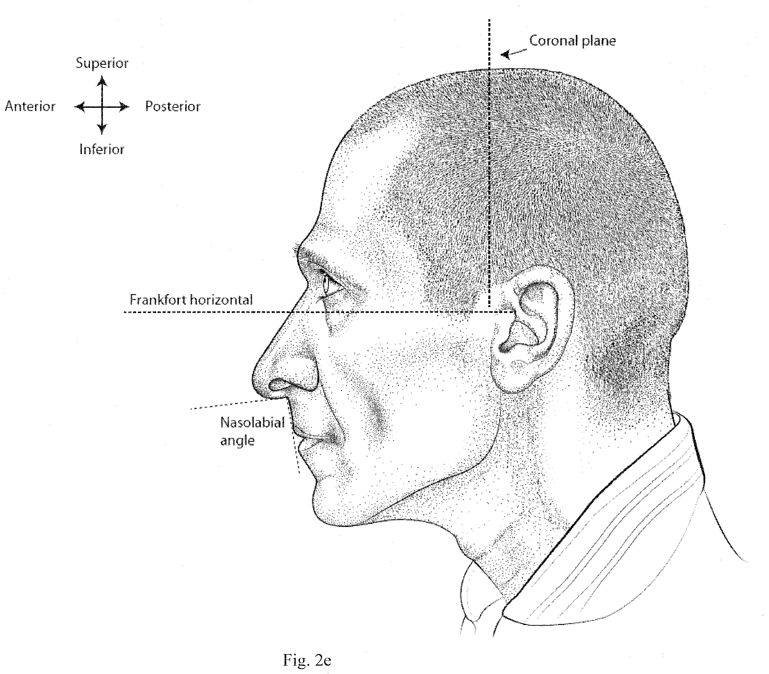

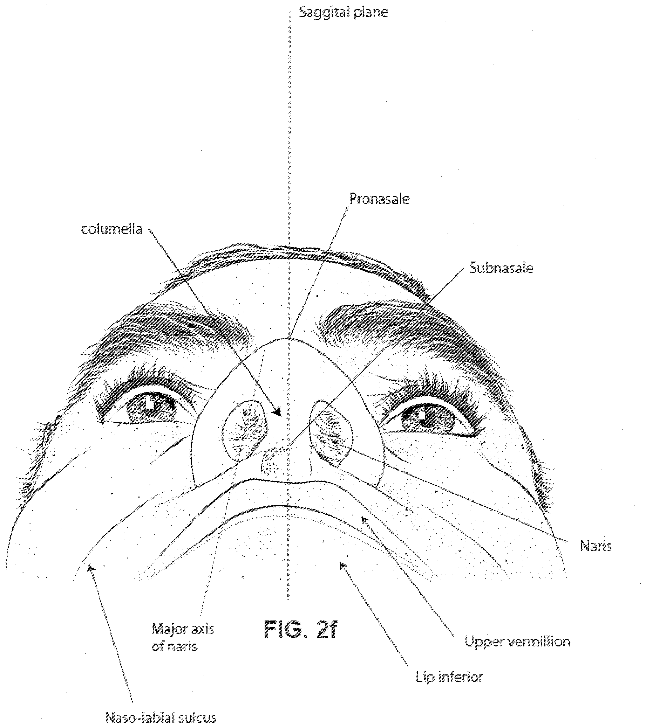

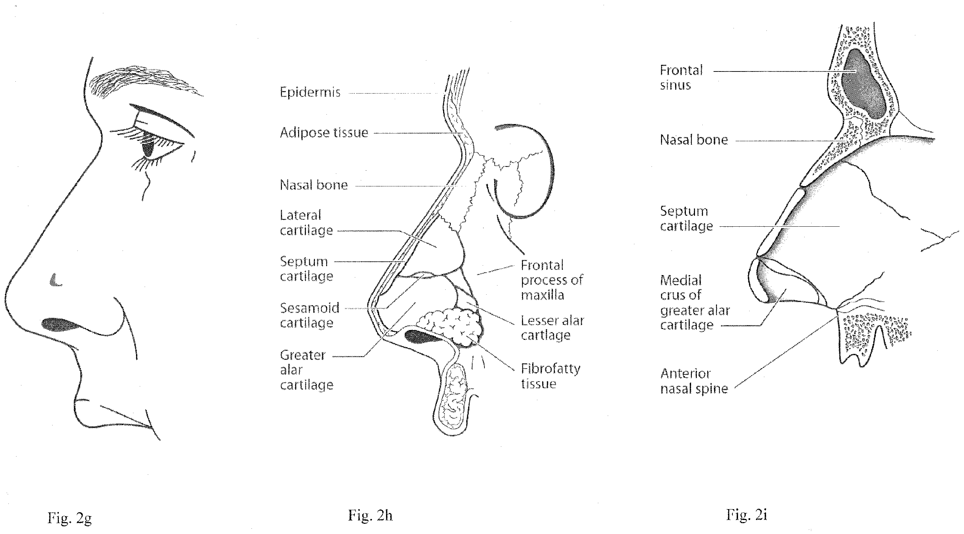

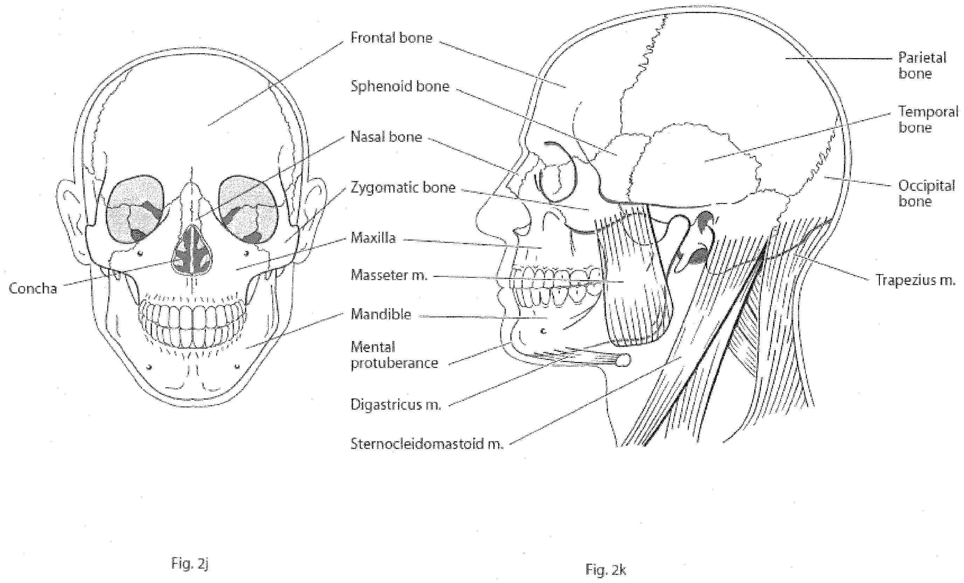

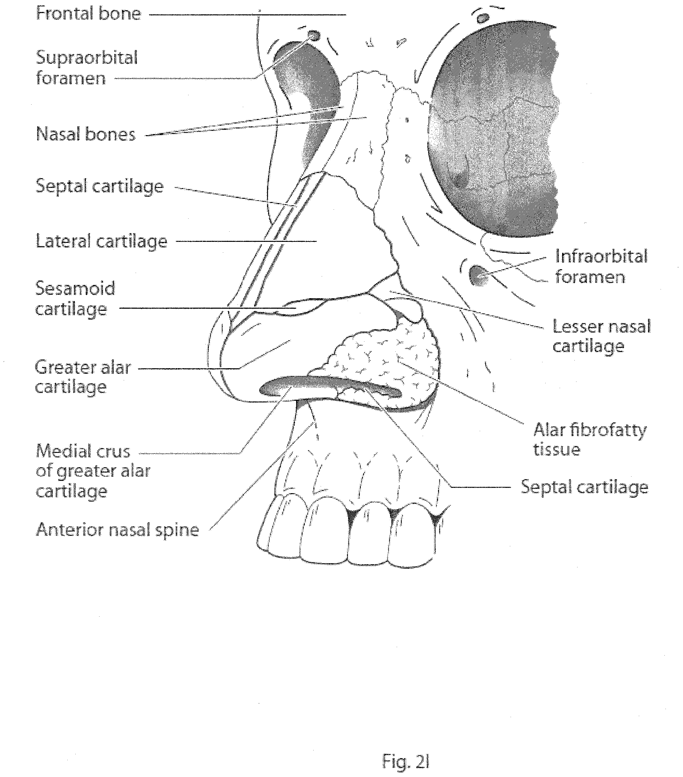

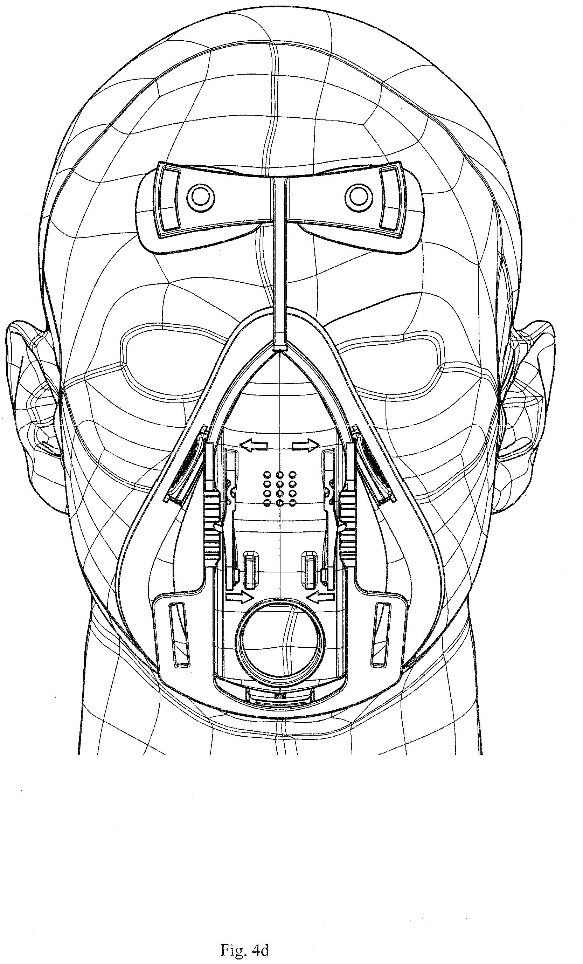

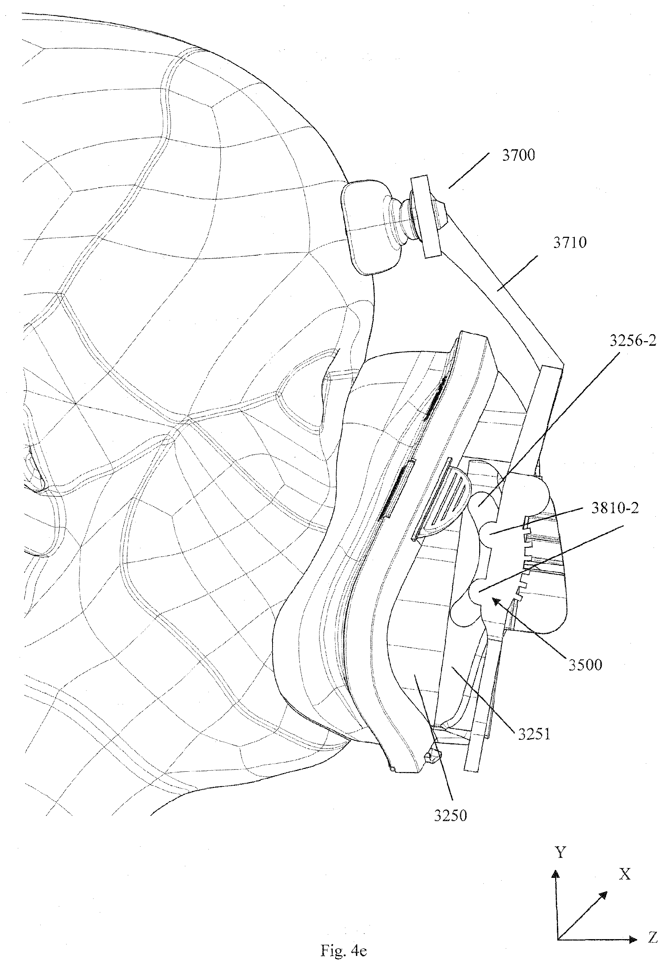

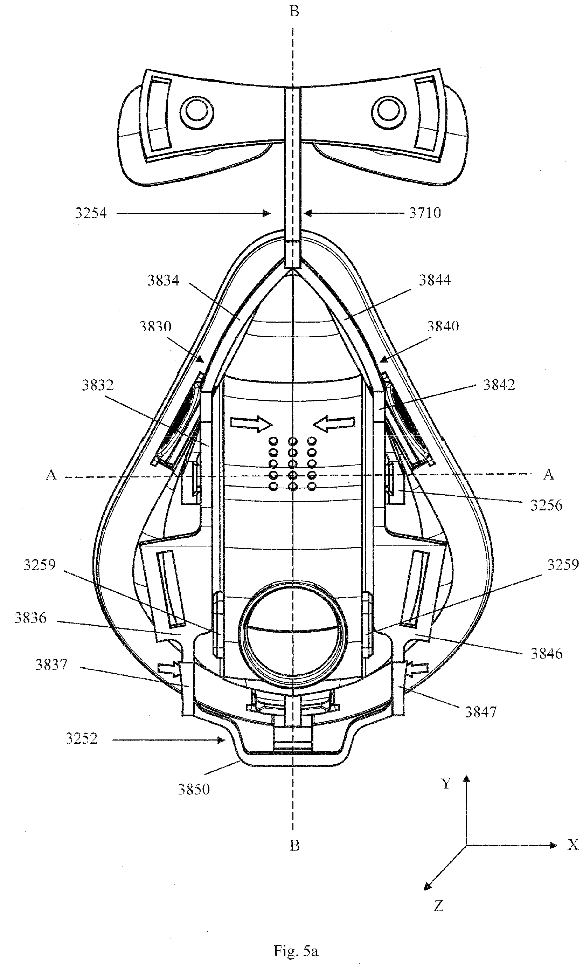

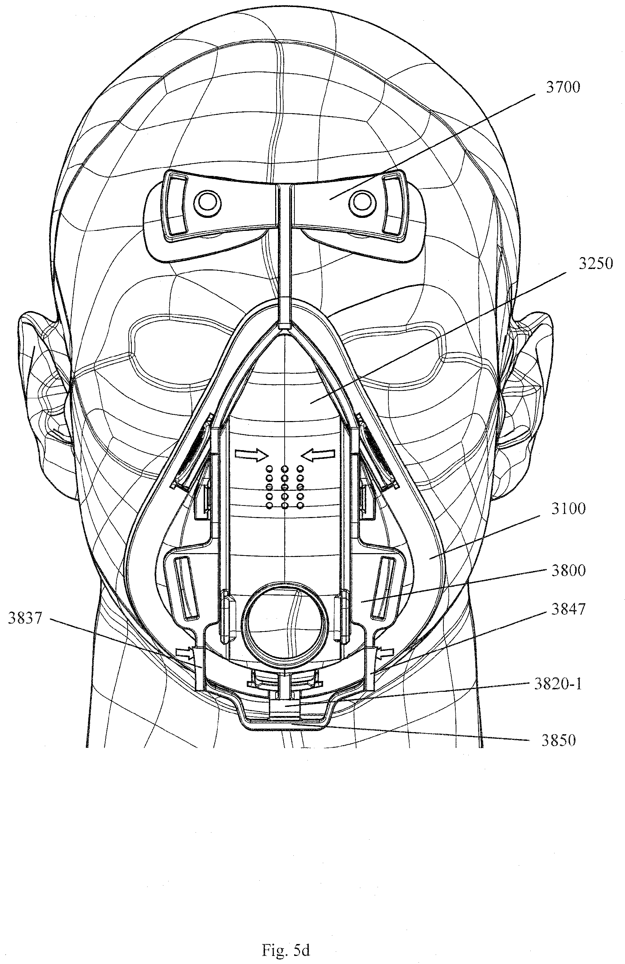

   Preferably, the frame is not integrally with the shell. The frame may be a separate component that can be removable connected to the shell. This may allow relative movement after assembly; i.e. movement in use and not only for the purpose of disassembly. This may be different from a frame being releasably connected to a shell whose position is fixed or determined after assembly.
2. The patient interface (3000) according to the preceding aspect, wherein the frame (3800) is adapted to be releasably coupled to the shell (3250).
3. The patient interface (3000) according to any one of the preceding aspects, wherein the shell is more rigid than the cushion.
4. The patient interface (3000) according to any one of the preceding aspects, wherein the shell (3250) is directly coupled to the frame (3800), preferably by a form fit and/or a press fit.
5. The patient interface (3000) according to any one of the preceding aspects, further comprising a forehead support (3700) adapted to be positioned against the forehead of a patient; and wherein the frame (3800) is coupled to the forehead support (3700).
   The forehead support may comprise one or more forehead parts which may be directly or indirectly connected to the frame.
6. The patient interface (3000) according to the preceding aspect, wherein the shell (3250) is indirectly coupled to the forehead support (3700) via the frame (3800).
7. The patient interface (3000) according to any one of the preceding aspects, wherein the relative movement of the shell (3250) with respect to the frame (3800) is a rotational movement.
8. The patient interface (3000) according to any one of the preceding aspects, wherein the relative movement of the shell (3250) with respect to the frame (3800) is an adjustably lockable movement.
   In other words, a relative position of the frame (and potentially the forehead support, if applicable) to shell is adjustable and may be locked in one or more adjusted position(s). In particular, the lockability may be provided in discrete and distinct positions; that is, in only a certain number of predetermined positions, such as in only 2, 3, 4, 5, 6, 7, 8, 9 or 10 positions. Alternatively, adjustability may be provided at infinite positions.
9. The patient interface (3000) according to any one of the preceding aspects, wherein the means providing the relative movement is preferably located between a lower end (3252) and an upper end (3254) of the shell (3250) and/or of the seal forming structure (3100).
   The upper and lower ends (3254, 3252) refer to the upper and lower ends of shell (3250) / seal forming structure (3100) in use when the wearer's head is in the upright position; i.e. in the longitudinal direction Y (compare, e.g., Fig. 4e) being generally parallel to the sagittal plane in use. By providing means for relative movement as defined above, such means may be positioned at a lower point of the mask than in the prior art. That is, in the prior art, such means - if at all known - are typically positioned at the forehead support while not allowing relative movement of frame shell but simply allowing for adjustment of the position of the forehead support at its upper end. By providing the means for relative movement as defined above, a configuration may be reached, which allows as more filigree, less bulky structure to be positioned in or close to the field of vision of the patient. The adjustment may thus also be positioned in an ergonomically favourable position to allow for easy single handed adjustment.
10. The patient interface (3000) according to the preceding aspect, wherein the means (3500, 3900) providing the relative movement is located approximately in the middle of the distance between the lower end (3252) and the upper end (3254) of the shell (3250) and/or of the seal forming structure (3100) and/or wherein the wherein the means (3500, 3900) providing the relative movement is located approximately in the lower nose/upper lip area when in the use position.
   In other words, the means may be provided approximately in the middle along the height of the mask when being worn in an upright position/wearing position. The above reference to upper and lower points of the seal forming structure, frame and or shell refers to the neutral state, i.e. when not worn by a patient.
11. The patient interface (3000) according to any of the 2 preceding aspects, wherein at least one connection portion (3256) of the shell (3250) and at least one connection portion (3810) of the frame (3800) form the means (3500, 3900) to allow the relative movement.
12. The patient interface (3000) according to any one of the preceding aspects, wherein the frame (3800) comprises two connection portions (3810) each coupling with respective connection portions (3256) of the shell (3250) to form a pair (3256, 3810), and wherein the pairs are laterally spaced apart.
   Laterally typically denotes a direction (X) perpendicular to the longitudinal axis B - B (compare, e.g., Figure 5a) and/or the sagittal plane of a patient wearing then mask. Put differently, laterally may denote a direction perpendicular to a symmetry plane of the mask or sideways as in the general use of the term when, e.g., seen in wearing position. As regards this symmetry plane, it is denoted that the mask may be substantially symmetric - that is, being symmetric apart from some structures. E.g. a mask may be provided with vent structures and/or additional ports that are only located in one location (or one side) of the mask. While such a mask would no longer be exactly symmetric, such a mask would still be considered substantially symmetric and have a respective plane of symmetry.
   Preferably, the pairs are laterally spaced apart far enough to allow a portion of the shell, containing the space housing the patient's nose tip, to fit between the two bars of the frame, thereby effectively limiting the overall size of the construct, and furthermore thereby also enabling the rotational axis to be located in the required position.
   Preferably, the pairs are laterally spaced apart by about 30mm to about 80mm.
13. The patient interface (3000) according to the preceding aspect, wherein at least a portion of the shell (3250) and/or of the plenum chamber (3200), preferably the portion of the shell (3250) and/or of the plenum chamber (3200) receiving at least partially the nose, is/are located between the two pairs.
   A patient interface according to that aspect may in particular be less obstructive. The patient interface less protruding from the wearer's face may further reduce the risk of unintentional displacement during the night when the patient moves. Positioning, ergonomics, and/or forces on the patients face, may thus be positioned. This also applies to other aspects of the invention.
14. The patient interface (3000) according to any one of the preceding aspects, wherein the frame (3800) comprises a first (3830) and a second (3840) bar being laterally spaced apart, wherein each bar (3830, 3840) comprises one connection portion (3810), wherein the bars (3830, 3840) each comprise a first portion (3832, 3842), and wherein the first portions (3832, 3842) extend generally parallel to the longitudinal axis (B-B) of the patient interface.
   The first portion may also be referred to as the upwardly extending portion or the upper portion of the bar. Furthermore, the longitudinal axis (B-B) is the axis of symmetry, is an axis parallel to the plane of symmetry. The first and second bars of the frame may by symmetrical.
15. The patient interface (3000) according to the preceding aspect, wherein the first and second bar (3830, 3840) each comprise a second portion (3834, 3844), the second portions (3834, 3844) connecting the first portions (3832, 3842) with a bar (3710) of the forehead support (3700).
16. The patient interface (3000) according to the preceding aspect, wherein the second portions (3834, 3844) and the bar (3710) of the forehead support form a generally Y-shaped pattern.
   When viewed in an upright position, as seen, e.g., in Fig. 5a, it may in particular be an inversely oriented Y-shaped pattern. It will be understood that the frame as shown in the preferred aspects has a grid like structure and may in total have (for example in a top view onto the mask as seen, e.g., in Figs. 5a, 4d, 5a, or 5d) comprise a V-shape, U-shape or oval-shape/O-shape. Preferably, the first and second bars as referred to above may be the sides of such U- or O-shape. Alternatively, the frame may take a more closed structure, with the respective bars being formed by, e.g., more voluminous or plate like portions and/or depressions or protrusions therefrom.
17. The patient interface (3000) according to any of the 2 preceding aspects, wherein the second portions (3834, 3844) are converging and join each other at a position located on or near the axis and/or plane of symmetry (B-B) of the patient interface (3000), and/or at or below the upper end of the shell (3250) and/or of the seal-forming structure (3100).
18. The patient interface (3000) according to any of the 3 preceding aspects, wherein the second portions (3834, 3844) are converging and, in use, join each other at a position in or adjacent the sagittal plane and/or in the region of the nose ridge or sellion.
   Preferably, the region comprises also slightly above or below the sellion, e.g. 2 cm above or below the sellion In particular, it may be preferable for the point of convergence as described above to be located towards the chin, e.g. below the nose ridge or sellion, thereby being less obstructive to the wearer's field of vision, e.g. the point of convergence may also be in the range of 1cm above to 3 cm below the sellion.
19. The patient interface (3000) according to any of the preceding aspects with the features of aspect 14, wherein the first and second bars (3830, 3840), particularly the first portions (3832, 3842) and/or the second portions (3834, 3844), are not (substantially) protruding in the lateral direction (x) further than the respective outer edge(s) or outer extent(s) of the shell (3250) and/or of the seal-forming structure (3100).
   Such a patient interface may be less obtrusive than others. Also, an advantageous distribution of forces is achieved. The non-protrusion as referred to in said aspect is particularly to be seen in a top view can be seen, e.g., in Figs. 5a, 4d, 5a, or 5d)
20. The patient interface (3000) according to any one of the preceding aspects, wherein a bar (3710) of the forehead support (3700) is located in the sagittal plane and/or in the axis/plane of symmetry (B-B).
21. The patient interface (3000) according to any of the preceding aspects with the features of aspect 14, wherein the first and second bar (3830, 3840) each comprise a third portion (3836, 3846) extending from the connecting portion (3810) downwardly.
   The third portion may also be referred to as the downwardly extending portion or lower portion of the bar. It may provide an easy grip and/or easy adjustment of the frame for better adaption to the face. It may be closed or open, e.g., providing a closed or open frame profile, such as, e.g., an O-shape or U-shape.
22. The patient interface (3000) according to the preceding aspect, wherein the third portions (3836, 3846) are connected via a connecting bar (3850) laterally connecting the first and second bar (3830, 3840), preferably at a lower end of the bars (3830, 3840).
   This feature may also help in providing an easy grip and increase rigidity. Such closed frame profile is also advantageous in that it provides for an opening or open area defined by the frame bars, through which a breathing lumen or a part thereof, such as an elbow, may extend and be connected to the shell.
23. The patient interface (3000) according to any of the 2 preceding aspects, wherein the third portions (3836, 3846) of the first and second bar (3830, 3840) each comprise an attachment point for a strap of the headgear (3300).
   This may lead to better adaptability and a more rigid design since the connecting portion (3837) transmits the tension forces applied to the attachment point of one bar directly to the attachment point of the other bar. Furthermore, it may also reduces the risk of unintended disassembly. According to a preferred aspect, four headgear connection portions are provided by the frame. Two headgear connections portions may be provided by the forehead support, preferably at its outer ends.
24. The patient interface (3000) according to any one of the preceding aspects, wherein the frame (3810) comprises at least one locking means (3820) adjustably engaging with at least one locking means (3258) of the shell (3250).
25. The patient interface (3000) according to the preceding aspect, wherein one of the locking means (3820, 3258) is configured as a gear rod or ratchet, and wherein the other of the locking means is configured as a protrusion or tongue adapted to interengage the gear rod or ratchet.
   The locking means can take other suitable mechanical interlock including snap-fit arrangement or the like.
26. The patient interface (3000) according to any of the preceding aspects with the features of aspects 22 and 24, wherein the locking means (3820) of the frame (3800) is located at the connecting bar (3850).
27. The patient interface (3000) according to any of the preceding aspects with the features of aspect 24, wherein the locking means (3820) of the frame (3800) is configured as a protrusion (3820-1), and wherein the locking means (3258) of the shell (3250) is configured as a gear rod or ratchet (3258-1).
   The locking means can take other suitable mechanical interlock structures allowing adjustable locking in a plurality of different positions, including snap-fit arrangements or the like.
28. The patient interface (3000) according to any of the preceding aspects with the features of aspects 14, 21 and 26, wherein the locking means (3820) located at the connecting bar (3850) is configured as a protrusion (3820-1), and wherein the connecting bar (3850) and the first and second bars (3830, 3840), preferably the third portions (3836, 3846) thereof, are adapted to flexibly bend, preferably so as to disengage the protrusion (3820-1) when the third portions (3836, 3846) are pressed laterally towards the axis/plane of symmetry (B-B).
   Thereby, adjustment of the position of the frame (3400) relative to the shell (3250) may be allowed. The disengagement of the protrusion may in particular be a disengagement from the, e.g. ratchet. In other words, the third portion is pressed laterally towards the axis/plane of symmetry (B-B) or pressed laterally inwardly, i.e. towards each other on this configuration.
29. The patient interface (3000) according to the preceding aspect, wherein the third portions (3836, 3846) are pressed laterally towards the axis/plane of symmetry (B-B) at predetermined, preferably surface increased, push areas (3837, 3847).
30. The patient interface (3000) according to the preceding aspect, wherein the shell (3250) comprises at least one, preferably two shoulders (3259) extending generally parallel to the third portions (3836, 3846), wherein the shoulders (3259) are located between the push areas (3837, 3847) and the connecting portions (3810); and wherein portions (3836-1, 3846-1) of the third portions (3836, 3846) adjacent the shoulders (3259) are adapted to abut against the shoulders when third portions are pressed laterally towards the axis of symmetry (B-B).
31. The patient interface (3000) with the features of aspect 14, wherein the frame (3810) comprises at least one, preferably two locking means (3820) located adjacent the connecting portion (3810) of the first and/or second bar(s) (3830, 3840).
32. The patient interface (3000) according to any of the preceding aspects with the features of aspect 25, wherein the locking means (3820) of the frame (3800) is/are configured as (a) gear rod(s) or ratchet structure (3820-2), and wherein the locking means (3258) of the shell (3250) comprise(s) the protrusion (3258-2) engaging the gear rod(s) (3820-2).
33. The patient interface (3000) according to the preceding aspect, wherein each protrusion (3258-2) is located at a resilient or bendable web portion (3258-3), the web portion (3258-3) being adapted to move the protrusion from an interlocked position to an non-interlocked position, preferably upon manual activation of a user.
   Exertion of a manual force may cause the locking means to disengage, while resilient or elastic forces cause engagement upon release of the manual force. Thereby adjustment of the frame (3800) relative to the shell (3250) may be allowed. With regard to the following aspects, it is to be noted that alternative aspects may cover kinematic exchange of positions and functionalities of those as expressed below while also other positions may be contemplated.
34. The patient interface (3000) according to the preceding aspect, wherein the bendable web portion(s) is/are integrally formed with the shell (3250).
35. The patient interface (3000) according to any of the 3 preceding aspects, wherein the protrusion(s) (3258-2) laterally engage the gear rod(s) (3820-2).
36. The patient interface (3000) according to any of the preceding aspects with the features of aspects 14 and 33, wherein the at least one, preferably two bendable web portion(s) (3258-3) is/are arranged adjacent and generally parallel to the first and/or second bar(s) (3830,3840), the bendable web portion(s) being adapted to bend towards the axis and/or plane of symmetry (B-B).
37. The patient interface (3000) according to any of the preceding aspects with the features of aspect 33, wherein the protrusion (3258-2) of the bendable web portion (3258-3) generally extends in the lateral direction (X).
38. The patient interface (3000) according to any of the preceding aspects, wherein the shell (3250) comprises two bendable web portions (3258-2) adapted to be operated by two fingers.
39. The patient interface (3000) according to any of the preceding aspects with the features of aspects 14 and 25, wherein the gear rod or ratchet extends from the outer surface of the first and/or second bar(s) (3830, 3840).
40. The patient interface (3000) according to the preceding aspect, wherein the gear rod or ratchet has and/or is located on a generally curved or acuate outer surface.
41. The patient interface (3000) according to any one of the preceding aspects, wherein the shell (3250) comprises at least two side walls (3251), preferably being oriented substantially parallel to each other.
42. The patient interface (3000) according to the preceding aspect, wherein the side wall(s) (3251) extend(s) substantially parallel to the first and/or second bar (3830, 3840).
43. The patient interface (3000) according to any of the preceding aspects with the features of aspects 14 and 41, wherein the first and/or second bar (3830, 3840), preferably at their connecting portions (3810) in use are arranged adjacent the side wall(s) (3251).
44. The patient interface (3000) according to any of the preceding aspects with the features of aspect 41, wherein the side wall (3251) is configured as a recessed or offset portion of the shell (3250).
45. The patient interface (3000) according to any of the preceding aspects with the features of aspect 41, wherein the side wall (3251) is a planar wall portion.
46. The patient interface (3000) according to any of the preceding aspects with the features of aspect 41, wherein the side wall (3251) forms a part of the walls limiting the plenum chamber (3200).
47. The patient interface (3000) according to any of the preceding aspects with the features of aspect 41, wherein the side walls (3251) are spaced apart in a lateral direction (X).
48. The patient interface (3000) according to any of the preceding aspects with the features of aspect 11, wherein the connection portion (3256) of the shell (3250) is configured as a groove (3256-2), preferably extending in the lateral direction (X) of the patient interface (3000).
   The groove may also be referred to as cam track, keyway or notch.
49. The patient interface (3000) according to any of the preceding aspects with the features of aspect 11, wherein the connection portion (3256) of the shell (3250) is configured as a hole (3256-1) preferably extending in the lateral direction (X) of the patient interface (3000).
   If located in the side wall, the hole (3256-1) may be an indentation or round or otherwise shaped recess.
50. The patient interface (3000) according to any one of the 2 preceding aspects, wherein the groove (3256-2) and/or the hole (3256-1) is/are located in the side wall (3251).
51. The patient interface (3000) according to any of the 3 preceding aspects, wherein the shell (3250) comprises at least one, preferably two protruding web portions (3253), wherein the hole(s) (3256-1) and/or groove(s) (3256-2) is/are located at the protruding web portion(s) (3253).
52. The patient interface (3000) according to the preceding aspect, wherein the protruding web portions (3253) generally protrude in a direction (Z) perpendicular to the lateral and longitudinal directions (X,Y).
   In other words, the portions extend in use in a direction away from the patient's face.
53. The patient interface (3000) according to any of the preceding aspects with the features of aspects of aspects 48 or 49 and 11, wherein the groove (3256-2) and/or the hole (3256-1) is/are adapted to be engaged by the connection portion (3810) of the frame (3800).
54. The patient interface (3000) according to any of the preceding aspects with the features of aspect 11, wherein at least one, preferably all connection portions (3810) of the frame (3800) is/are configured as a protrusion (3810-1) or a plurality of protrusions (3810-2) preferably extending in the lateral direction (X) of the patient interface (3000).
   The protrusions may also be referred to as lateral protrusion or shafts. The protruding portion may be a cam, preferably designed for mechanical interengagement with the groove or hole, and particularly for movement relative thereto, such as turning or pivoting or rotating in the hole or such as sliding along the groove.
55. The patient interface (3000) according to any of the preceding aspects with the features of aspects 48 or 49 and 54, wherein the groove (3256-2) and/or the hole (3256-1) and the protrusions (3810-1, 3810-2) are adapted to engage and/or disengage upon bending of the frame (3800) in the lateral direction of the patient interface (3000).
   This may ease assembly and cleaning of the interface.
56. The patient interface (3000) according to any of the preceding aspects with the features of aspect 14, wherein the frame (3800), preferably the first and second bars (3830, 3840), and most preferably the first and/or second portions (3832, 3842; 3834, 3844) of the first and second bars (3830, 3840) are adapted to bend, preferably when a lateral force (F) is applied to the first and/or second portions (3832, 3842; 3834, 3844).
57. The patient interface (3000) according to any of the preceding aspects with the features of aspects 14, 48 or 49 and 54, wherein the groove (3256-2) and/or the hole (3256-1) is/are located in the side wall (3251), and wherein the protrusions (3810-1, 3810-2) are adapted to engage and/or disengage the groove (3256-2) and/or the hole (3256-1) when the first and/or second portions (3832, 3842; 3834, 3844) are pressed in a lateral direction (X) away from each other, preferably by applying a laterally outwardly oriented force (F) to grips (3833, 3843) preferably located at the first portions (3832, 3842).
58. The patient interface (3000) according to any of the preceding aspects with the features of aspects 14 and 51, wherein the groove (3256-2) and/or the hole (3256-1) is/are located in the protruding web portion(s) (3253), and wherein the protrusions (3810-1, 3810-2) are adapted to engage and/or disengage the groove (3256-2) and/or the hole (3256-1) when the first and/or second portions (3832, 3842; 3834, 3844) are pressed in a lateral direction (X) towards each other, preferably by applying a laterally inwardly oriented force (F) to grips (3833, 3843) preferably located at the first portions (3832, 3842).
   The fulcrum as defined in aspect 1 may be referred to as a rotation axis wherein the rotation axis is displaced from the entity adapted to move rotationally. The curved path may in particular be a circular or elliptical arc.
61. The patient interface (3000) according to any of the preceding aspects with the features of aspect 48 and 54, wherein the shell (3250) and the frame (3800) are coupled via the at least one groove (3256-2) of the shell (3250) and the at least one protrusion (3810-2) of the frame preferably extending in the lateral direction (X) of the patient interface (3000).
   This may allow a relative movement of the shell (3250) with respect to the frame (3800).
62. The patient interface (3000) according to any of the preceding aspects with the features of aspect 48, wherein the at least one groove (3256-2) has a generally curved path, preferably so that the frame (3800) follows a generally curved path.
63. The patient interface (3000) according to any of the preceding aspects with the features of aspect 14, wherein two grooves (3256-2) having a curved path are located on two side walls (3251) of the shell (3250), each groove (3256-2) being engaged by at least one, preferably two laterally extending protrusions (3810-2) of the first and second bars (3830, 3840) located adjacent the side walls (3251), wherein the protrusions (3810-2) are adapted to slide inside the grooves (3256-2), preferably such that the frame (3800) follows a generally curved path.
64. The patient interface (3000) according to any of the preceding aspects with the features of aspect 48, wherein one laterally extending protrusion (3810-2) engages each groove (3256-2), the laterally extending protrusion (3810-2) having a curved shape that correspond to the curvature of the groove.
65. The patient interface (3000) according to any of the preceding aspects with the features of aspect 48, wherein a plurality of laterally extending protrusion (3810-2) engages each groove (3256-2), the plurality of laterally extending protrusion (3810-2) having a generally round shape and being located on a curved path corresponding to the curved path of the groove (3256-2).
   In particular, corresponding curved paths may have substantially the same fulcrum.
   The joint as defined in aspect 1 may also be referred to as a hinge, a pivot, or an articulated joint.
67. The patient interface (3000) according to any of the preceding aspects with the features of aspect 49 and 54, wherein the shell (3250) and the frame (3800) are coupled via the at least one hole (3256-1) of the shell (3250) and the at least one protrusion (3810-1) of the frame preferably extending in the lateral direction (X) of the patient interface (3000).
   This may allow a relative movement of the shell (3250) with respect to the frame (3800).
68. The patient interface (3000) according to any of the preceding aspects with the features of aspect 14, wherein two holes (3256-1) are located in two protruding web portions (3253) of the shell (3250), each hole (3256-1) being engaged by one laterally extending protrusion (3810-1) of the first and second bars (3830, 3840) located adjacent the protruding web portions (3253), wherein each protrusion (3810-2) is adapted to rotate inside the hole (3256-1).
69. The patient interface (3000) according to any of the preceding aspects with the features of aspects 60 or 66, wherein the rotation axis (A-A) of the joint (3900) and/or the fulcrum (A'-A') of the sliding arrangement (3500) extend(s) perpendicular to the longitudinal axis (B-B) of the patient interface (3000) in a lateral direction (X).
70. The patient interface (3000) according to any of the preceding aspects, wherein the longitudinal axis (B-B) is the axis of symmetry or is included in the plane of symmetry.
71. The patient interface (3000) according to any of the preceding aspects with the features of aspects 60 or 66, wherein the rotation axis (A-A) and/or the fulcrum (A'-A') of the sliding arrangement (3500) extend(s) through the plenum chamber (3200), the shell (3250) and/or the seal-forming structure (3100).
72. The patient interface (3000) according to any of the preceding aspects with the features of aspects 66, wherein the rotation axis (A-A) is located in the application position outwardly offset from plenum chamber (3200).
   In other words, the rotation axis is offset in a direction Z away from the patient (indicated in Fig. 2e). I.e. in a direction generally orthogonal to longitudinal and lateral directions X, Y.
73. The patient interface (3000) according to any of the preceding aspects with the features of aspects 60 or 66, wherein the rotation axis (A-A) of the joint (3900) and/or the fulcrum (A'-A') of the sliding arrangement (3500) is/are located between an lower end (3252) and an upper end (3254) of the shell (3250) and/or of the seal-forming structure (3100) and/or wherein the rotation axis (A-A) of the joint (3900) and/or the fulcrum (A'-A') of the sliding arrangement (3500) is/are located approximately in the lower nose/upper lip area when used by a patient.
   Upper and lower ends (3252, 3254) refer to the upper and lower ends of the shell 3250 in use; i.e. in the longitudinal direction Y.
74. The patient interface (3000) according to any of the preceding aspects with the features of aspects 60 or 66, wherein, in use, the rotation axis (A-A) of the joint (3900) and/or the fulcrum (A'-A') of the sliding arrangement (3500) is/are located in a vertical direction (Y) between the sellion and the mouth of a patient, preferably at the nasal ridge, pronasale or lip superior.
75. The patient interface (3000) according to any of the preceding aspects with the features of aspect 66, wherein, in use, the rotation axis (A-A) of the joint (3900) is located in an offset distance from the face of the patient of about 2 cm to about 10 cm, preferably of about 3 cm to about 6 cm, and most preferably of about 4,5 cm.
   The offset is in particular in the direction Z away from the patient's face.
76. The patient interface (3000) according to any of the preceding aspects with the features of aspect 60, wherein, in use, the fulcrum (A'-A') of the sliding arrangement (3500) is located in an [outwardly oriented] offset distance from the face of the patient of about - 2 cm to about 8 cm, preferably of about 0 cm to about 3 cm, and most preferably of about 1,5 cm.
   That is, -2 cm is a negative offset; i.e. fulcrum located at a distance of 2 cm oriented inwardly.
   In other words, as regards aspect 1, the pivoting point or hinge is positioned at a centre of gravity or alternatively the centroid of the mask. This may allow for adjustment without having an impact on the seal. Adjacent may mean that the respective two structures are displaced by no more than 4 cm, 3 cm, 2 cm, 1 cm or 0,5 cm from one another.
   Positioning the rotational axis or fulcrum point close to or at a centre of gravity or the centroid of the mask may be advantageous in that forces resulting from therapy pressure or headgear may not unintentionally move the mask out of its present orientation relative to the frame as the patient releases the interlock mechanism in an attempt to re-position the mask. Thus, the mask may be easier and/or more intuitive to adjust by the patient than other examples of the prior art. This may, e.g., become of increasing importance in applications where therapy pressures supplied by the system are approaching the upper end of the therapeutic range.
78. The patient interface (3000) according to any of the preceding aspects, wherein the patient interface (3000) is adapted for delivering breathable gas at positive pressure, preferably for a PAP treatment.
79. The patient interface (3000) according to any of the preceding aspects,
   wherein the patient interface (3000) comprises a means for adjustably adapting the relative position of the frame (3800) to the forehead support (3700).
80. The patient interface (3000) according to any of the preceding aspects with the features of aspect 60, wherein, in use, the seal forming structure (3100) contacts the patent inter alia at
   an uppermost sealing point located, in use, when the patient interface (3000) and the patient's head are in upright positions, at the uppermost end of the seal forming structure (3100), and
   a lowest sealing point located, in use, when the patient interface (3000) and the patient's head are in upright positions, at the lowest end of the seal forming structure (3100); and
   wherein a frontal plane (E1) is defined
   to include the uppermost sealing point and the lowest sealing point and
   to be perpendicular to the saggital plane of the patient;
   wherein a perpendicular plane (E2) is defined
   to be perpendicular to the saggital plane and to the frontal plane (E1) and
   to include the center of mass of a sub structure being composed of the seal forming structure (3100) and the shell (3250);
   wherein the fulcrum (A'-A') is located within a range of 4 cm from the perpendicular plane (E2).
81. The patient interface (3000) according to the preceding aspect, wherein the fulcrum (A'-A') is located within a range of 3 cm, 2 cm, 1 cm or 5 mm from the perpendicular plane (E2) or is located within the perpendicular plane (E2).
82. The patient interface (3000) according to any of the preceding aspects with the features of aspect 80, wherein the fulcrum (A'-A') is located at a distance in a range from 0.5 cm to 10 cm, preferably 1 cm to 8 cm, such as 3 cm to 6 cm, from the center of mass of the sub structure being composed of the seal forming structure (3100) and the shell (3250).
   For the sake of determining the center of mass of this sub structure, every portion formed integrally with the shell and/or the seal forming structure and every portion connecting these two should be construed to be a part of this sub structure
   This may also be a reference point to where theoretically the forces resulting from therapy pressure are applied, i.e. a point defined as the projected maximum cross-sectional area of the plenum chamber's interior.
83. The patient interface (3000) according to any of the preceding aspects with the features of aspect 80, wherein the uppermost sealing point is the nasal bridge of the patient.
84. The patient interface (3000) according to any of the preceding aspects with the features of aspect 80, wherein the lowest sealing point is the chin of the patient.
85. The patient interface (3000) according to any of the preceding aspects with the features of aspect 66, wherein, in use, the seal forming structure (3100) contacts the patient inter alia at
   an uppermost sealing point located, in use, when the patient interface (3000) and the patient's head are in upright positions, at the uppermost end of the seal forming structure (3100), and
   a lowest sealing point located, in use, when the patient interface (3000) and the patient's head are in upright positions, at the lowest end of the seal forming structure (3100); and
   wherein a frontal plane (E1) is defined
   to include the uppermost sealing point and the lowest sealing point and
   to be perpendicular to the saggital plane of the patient;
   wherein a perpendicular plane (E2) is defined
   to be perpendicular to the saggital plane and to the frontal plane (E1) and
   to include the center of mass of a sub structure being composed of the seal forming structure (3100) and the shell (3250);
   wherein the rotation axis (A-A) is located within a range of 4 cm from the perpendicular plane (E2).
86. The patient interface (3000) according to the preceding aspect, wherein the rotation axis (A'-A') is located within a range of 3 cm, 2 cm, 1 cm or 5 mm from the perpendicular plane (E2) or is located within the perpendicular plane (E2).
87. The patient interface (3000) according to any of the preceding aspects with the features of aspect 85, wherein the rotation axis (A-A) is located within a range of 4 cm, 3 cm, 2 cm, 1 cm or 5 mm from the center of mass of the sub structure being composed of the seal forming structure (3100) and the shell (3250) or is located at this center of mass.
88. The patient interface (3000) according to any of the preceding aspects with the features of aspect 85, wherein the uppermost sealing point is the nasal bridge of the patient.
89. The patient interface (3000) according to any of the preceding aspects with the features of aspect 85, wherein the lowest sealing point is the chin of the patient.
   The location of the fulcrum and/or rotational axis as described above is particularly advantageous in that the seal-forming structure (e.g. mask cushion) and plenum chamber are not biased towards a certain direction, as is usually the case in present masks. This means that in case of an imbalance in the system, the mask system will have a tendency to "jump" into a neutral position where forces are in equilibrium, as soon as the interlock between frame and shell is released. Such an imbalance can for example be caused by external forces acting on the membrane, e.g. due to excessive headgear tension, or by faces outside of the average requiring different angles between frame and shell. Effectively, due to these self-adjusting properties, the mask may be more intuitive to use and/or require less force to achieve a seal in all areas of the face. It is again pointed out that positioning the rotational axis or fulcrum point close to or at a centre of gravity or the centroid of the mask may be advantageous in that forces resulting from therapy pressure or headgear may not unintentionally move the mask out of its present orientation relative to the frame as the patient releases the interlock mechanism in an attempt to re-position the mask. Thus, the mask may be easier and/or more intuitive to adjust by the patient than other examples of the prior art. This may, e.g., become of increasing importance in applications where therapy pressures supplied by the system are approaching the upper end of the therapeutic range.
90. The patient interface (3000) according to any of the preceding aspects with the features of aspect 60, wherein the seal-forming structure (3100) defines an interior area (4000) having a geometric centre and wherein a distance in the longitudinal direction (Y) between the geometric centre of the interior area (4000) and the fulcrum (A'-A') is below 4cm.
91. The patient interface (3000) according to the preceding aspect, wherein the distance in the longitudinal direction (Y) between the geometric centre of the interior area (4000) and the fulcrum (A'-A') is below 3cm, preferably below 2cm, further preferably below 1 cm, still further preferably below 0,5 cm and most preferably below 0,2 cm.
92. The patient interface (3000) according to any of the preceding aspects with the features of aspect 66, wherein the seal-forming structure (3100) defines an interior area (4000) having a geometric centre and wherein a distance in the longitudinal direction (Y) between the geometric centre of the interior area (4000) and the rotation axis (A'-A') is below 4cm.
93. The patient interface (3000) according to the preceding aspect, wherein the distance in the longitudinal direction (Y) between the geometric centre of the interior area (4000) and the rotation axis (A'-A') is below 3cm, preferably below 2cm, further preferably below 1 cm, still further preferably below 0,5 cm and most preferably below 0,2 cm.
94. The patient interface (3000) according to any of the preceding aspects with the features of aspect 60, wherein the mask plenum chamber (3200) defines a projected area in the longitudinal and lateral direction, the projected area having a geometric centre and wherein a distance in the longitudinal direction (Y) between the geometric centre of the projection area (4000) and the fulcrum (A'-A') is below 4cm.
95. The patient interface (3000) according to the preceding aspect, wherein the distance in the longitudinal direction (Y) between the geometric centre of the projected area and the fulcrum (A'-A') is below 3cm, preferably below 2cm, further preferably below 1 cm, still further preferably below 0,5 cm and most preferably below 0,2 cm.
96. The patient interface (3000) according to any of the preceding aspects with the features of aspect 66, wherein the mask plenum chamber (3200) defines a projected area in the longitudinal and lateral direction, the projected area having a geometric centre and wherein a distance in the longitudinal direction (Y) between the geometric centre of the projection area and the rotation axis (A'-A') is below 4cm.
97. The patient interface (3000) according to the preceding aspect, wherein the distance in the longitudinal direction (Y) between the geometric centre of the projected area and the rotation axis (A'-A') is below 3cm, preferably below 2cm, further preferably below 1 cm, still further preferably below 0,5 cm and most preferably below 0,2 cm.

Thus, a patient interface being well balanced with regard to the force resulting from the pressure supplied via the patient interface to the patient may be provided. The longitudinal direction corresponds to the vertical direction when the patient interface is worn and the wearer stands in an upright position. The lateral direction corresponds to the direction defining "left" and "right" when the patient interface is worn and the wearer stands in an upright position. The patient interface of the invention has numerous advantages. E.g. by providing the means providing the relative movement between the upper end and the lower end of the shell and/or close to the center of mass of the patient interface, a better adjustability to face geometry can be reached than is possible when the means are provided at upper or lower ends. Furthermore, provision of the means at this location may not require such means to be positioned in the field of vision of the user, thereby improving patient comfort. Further still, the invention may be simpler to adjust and more intuitive to use and provide a better fit, resulting in a better seal, requiring less tensioning of the patient interface. Further advantages may include:
less parts,
simple adjustability, such as with one hand only, and
simple assembly and disassembly.

Other features of the invention will be apparent from consideration of the information contained in the following detailed description, abstract, drawings and claims.

### 3 BRIEF DESCRIPTION OF THE DRAWINGS

The invention is illustrated by way of example, and not by way of limitation, in the figures of the accompanying drawings, in which like reference numerals refer to similar elements including:

### 3.1 TREATMENT SYSTEMS

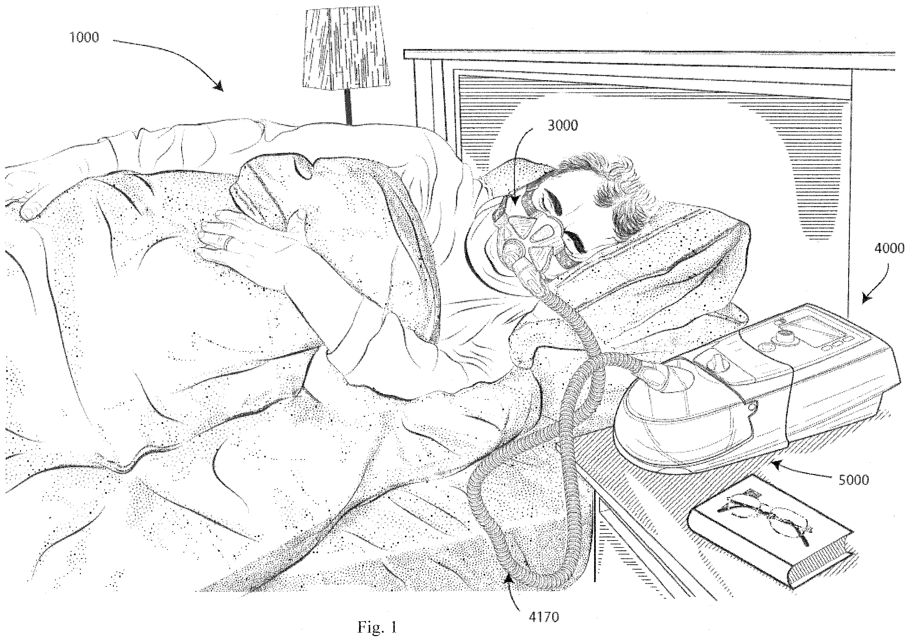

Fig. 1 shows a system including a patient 1000 wearing a patient interface 3000, preferably in the form of a full-face mask, receiving a supply of air at positive pressure from an RPT device 4000. Air from the RPT device is humidified in a humidifier 5000, and passes along an air circuit 4170 to the patient 1000.

### 3.2 RESPIRATORY SYSTEM AND FACIAL ANATOMY

Fig. 2a shows an overview of a human respiratory system including the nasal and oral cavities, the larynx, vocal folds, oesophagus, trachea, bronchus, lung, alveolar sacs, heart and diaphragm.
Fig. 2b shows a view of a human upper airway including the nasal cavity, nasal bone, lateral nasal cartilage, greater alar cartilage, nostril, lip superior, lip inferior, larynx, hard palate, soft palate, oropharynx, tongue, epiglottis, vocal folds, oesophagus and trachea.
Fig. 2c is a front view of a face with several features of surface anatomy identified including the lip superior, upper vermilion, lower vermilion, lip inferior, mouth width, endocanthion, a nasal ala, nasolabial sulcus and cheilion. Also indicated are the directions superior, inferior, radially inward and radially outward.
Fig. 2d is a side view of a head with several features of surface anatomy identified including glabella, sellion, pronasale, subnasale, lip superior, lip inferior, supramenton, nasal ridge, alar crest point, otobasion superior and otobasion inferior. Also indicated are the directions superior & inferior, and anterior & posterior.
Fig. 2e is a further side view of a head. The approximate locations of the Frankfort horizontal and nasolabial angle are indicated. The coronal plane is also indicated.
Fig. 2f shows a base view of a nose with several features identified including naso-labial sulcus, lip inferior, upper Vermilion, naris, subnasale, columella, pronasale, the major axis of a naris and the sagittal plane.
Fig. 2g shows a side view of the superficial features of a nose.
Fig. 2h shows subcutaneal structures of the nose, including lateral cartilage, septum cartilage, greater alar cartilage, lesser alar cartilage, sesamoid cartilage, nasal bone, epidermis, adipose tissue, frontal process of the maxilla and fibrofatty tissue.
Fig. 2i shows a medial dissection of a nose, approximately several millimeters from a sagittal plane, amongst other things showing the septum cartilage and medial crus of greater alar cartilage.
Fig. 2j shows a front view of the bones of a skull including the frontal, nasal and zygomatic bones. Nasal concha are indicated, as are the maxilla, and mandible.
Fig. 2k shows a lateral view of a skull with the outline of the surface of a head, as well as several muscles. The following bones are shown: frontal, sphenoid, nasal, zygomatic, maxilla, mandible, parietal, temporal and occipital. The mental protuberance is indicated. The following muscles are shown: digastricus, masseter, sternocleidomastoid and trapezius.
Fig. 2I shows an anterolateral view of a nose.

### 3.3 PATIENT INTERFACE

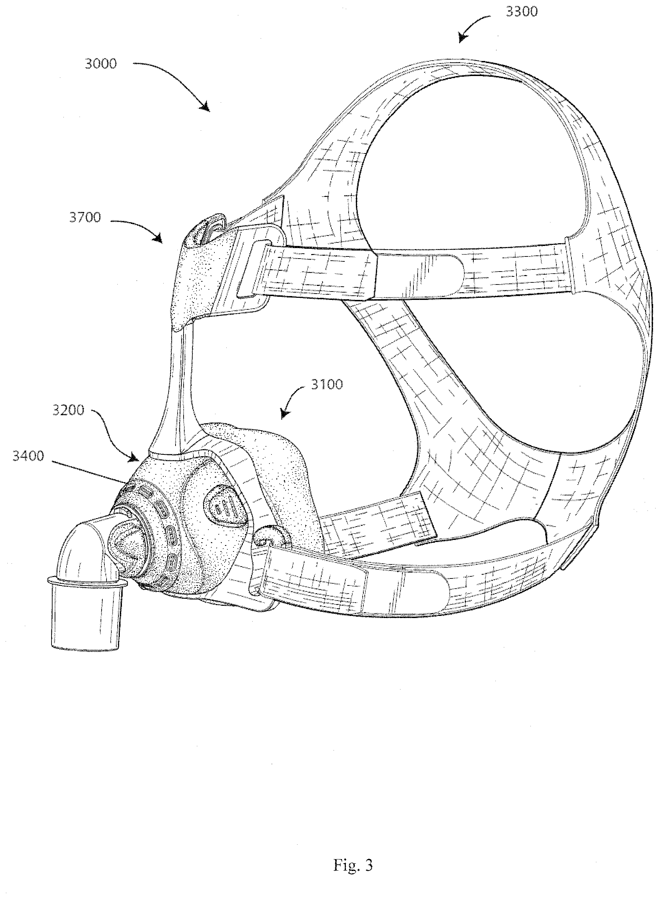

Fig. 3 shows a patient interface in the form of a nasal mask.

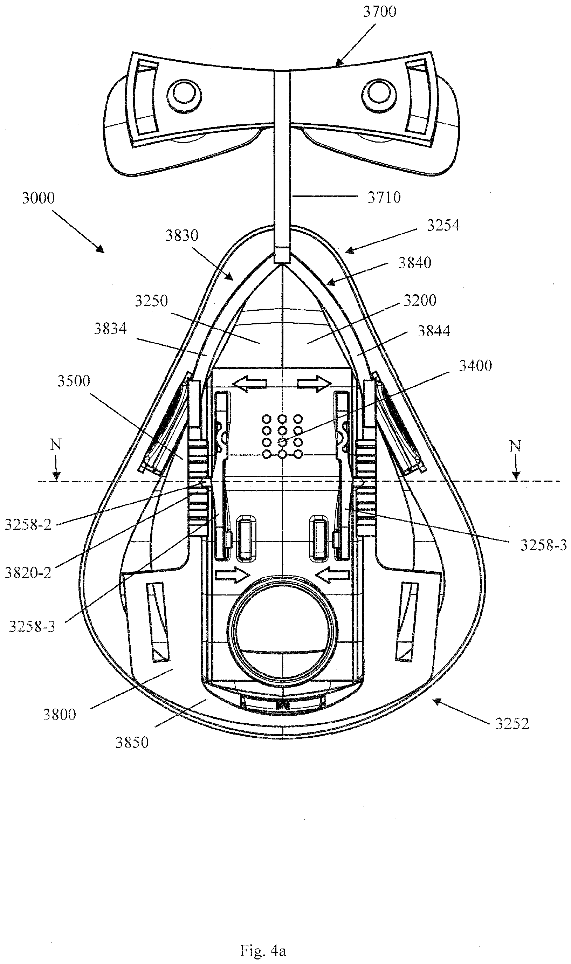

Fig. 4a a front view of a patient interface 3000,

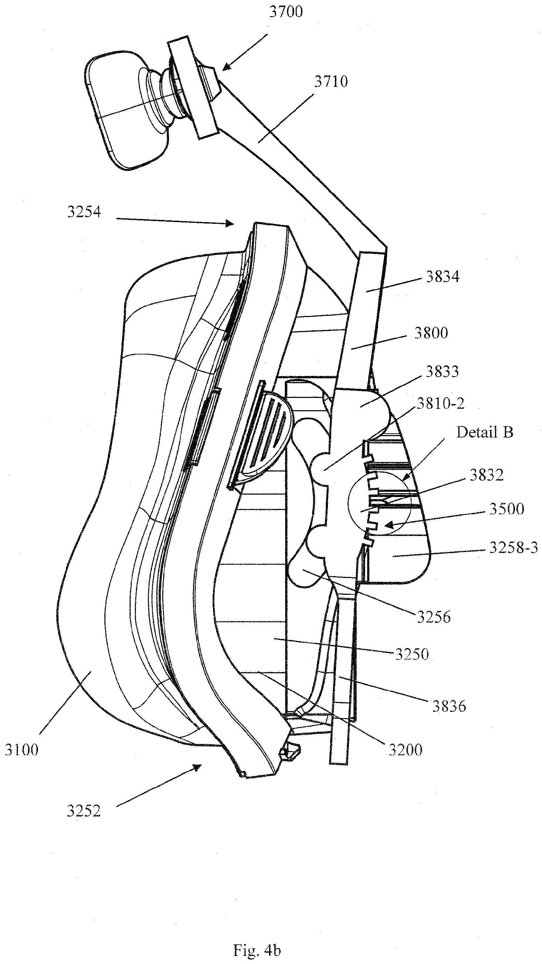

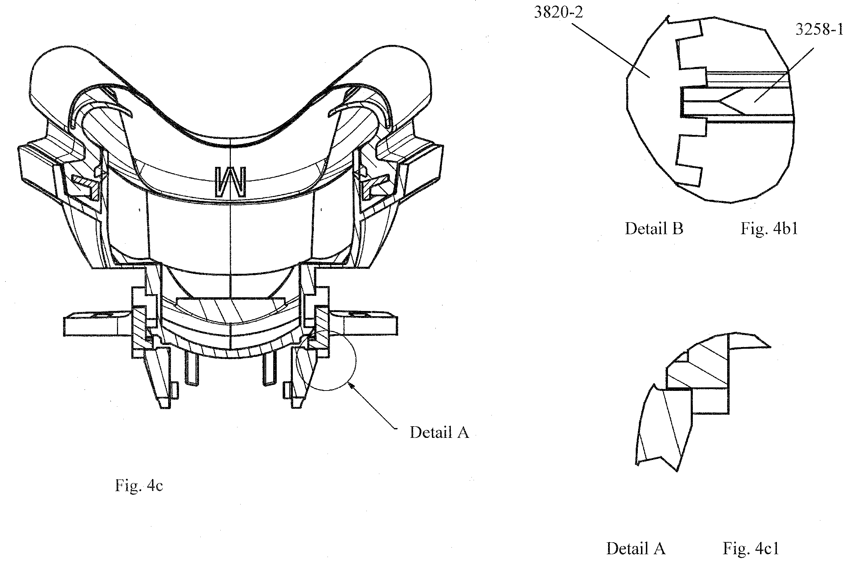

Fig. 4b a side view of a patient interface 3000,
Fig. 4b1 an enlarged detail of Fig. 4b,
Fig. 4c a cross-sectional view of a patient interface 3000 along the line N - N in Fig. 4a,
Fig. 4c1 an enlarged detail of Fig. 4c,
Fig. 4d a front view of a patient interface 3000 with a user,
Fig. 4e a side view of a patient interface 3000 with a user,
Fig. 5a a front view of a patient interface 3000,

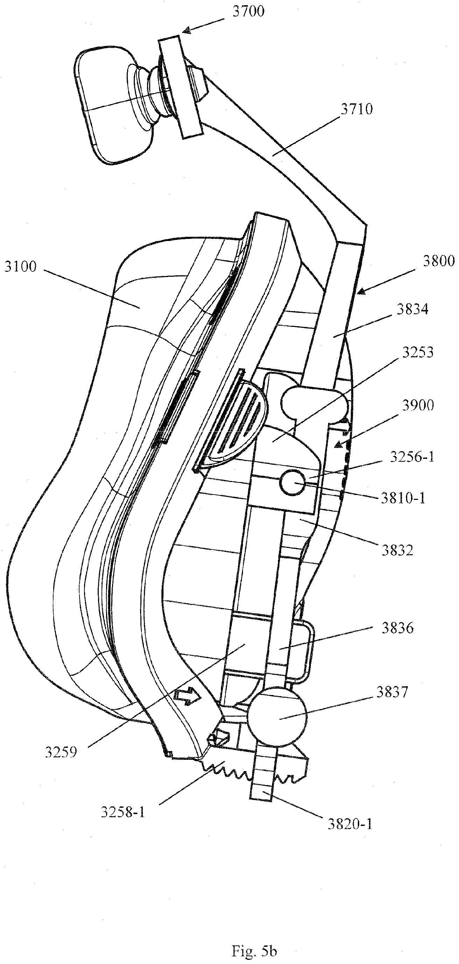

Fig. 5b a side view of a patient interface 3000,

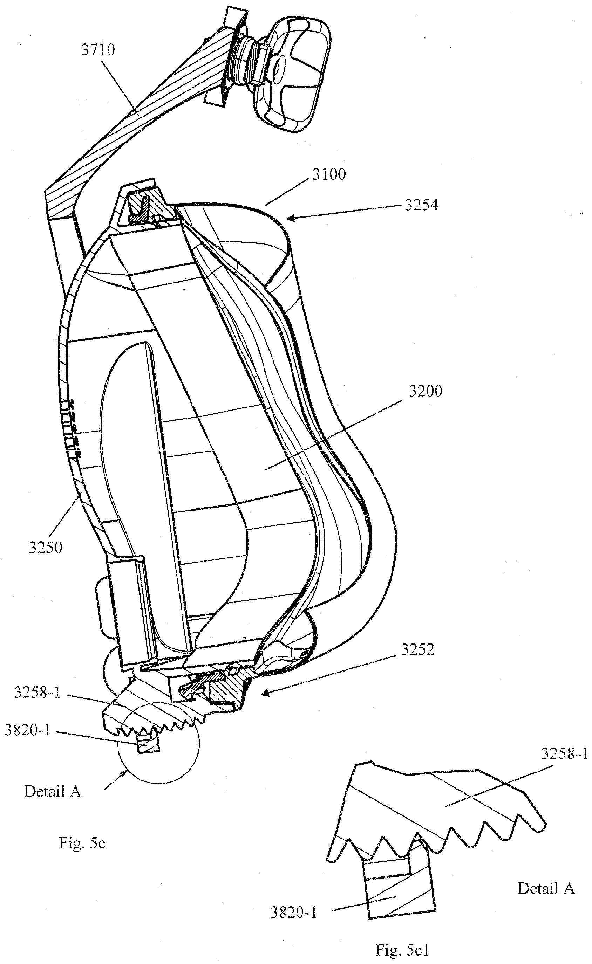

Fig. 5c a cross-sectional view along the line B-B of a patient interface 3000,
Fig. 5c1 an enlarged detail of Fig. 5c,
Fig. 5d a front view of a patient interface 3000 with a user,

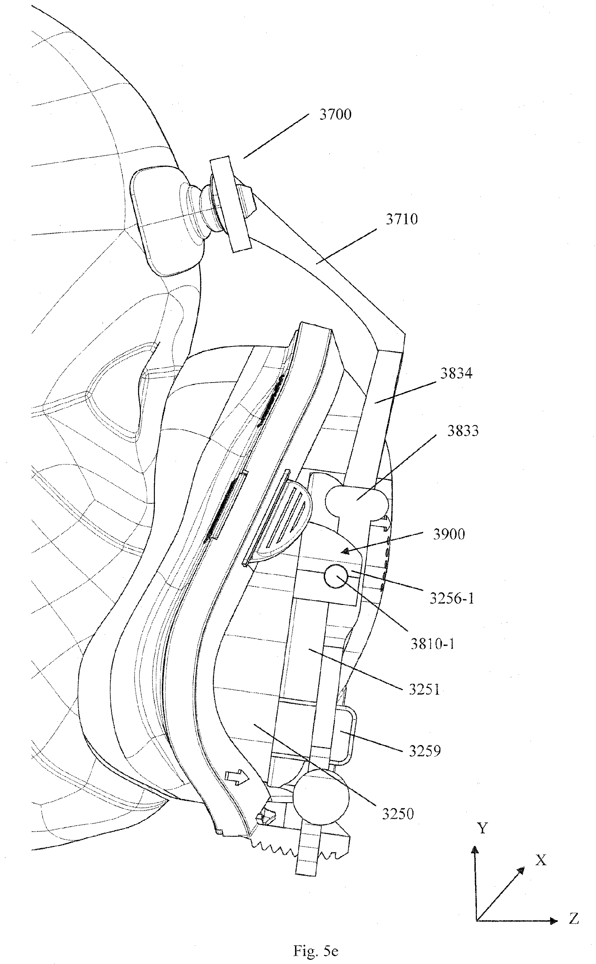

Fig. 5e a side view of a patient interface 3000 with a user,

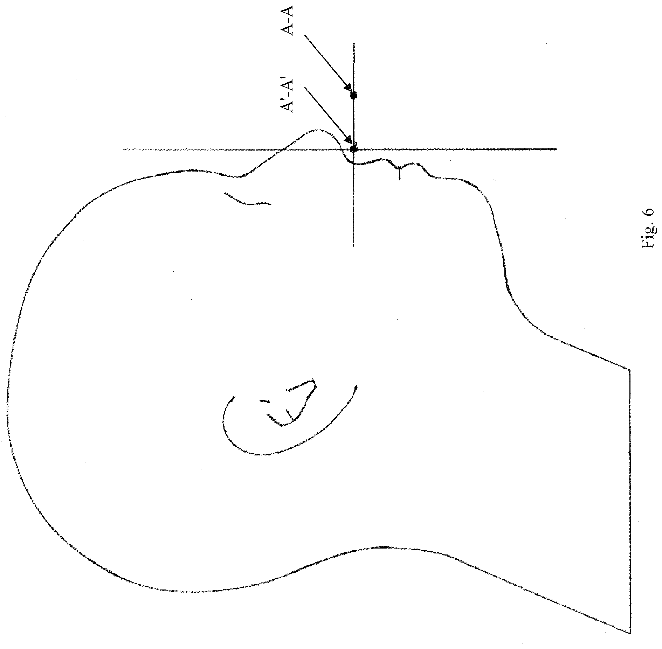

Fig. 6a a side view indicating the axis of rotation,

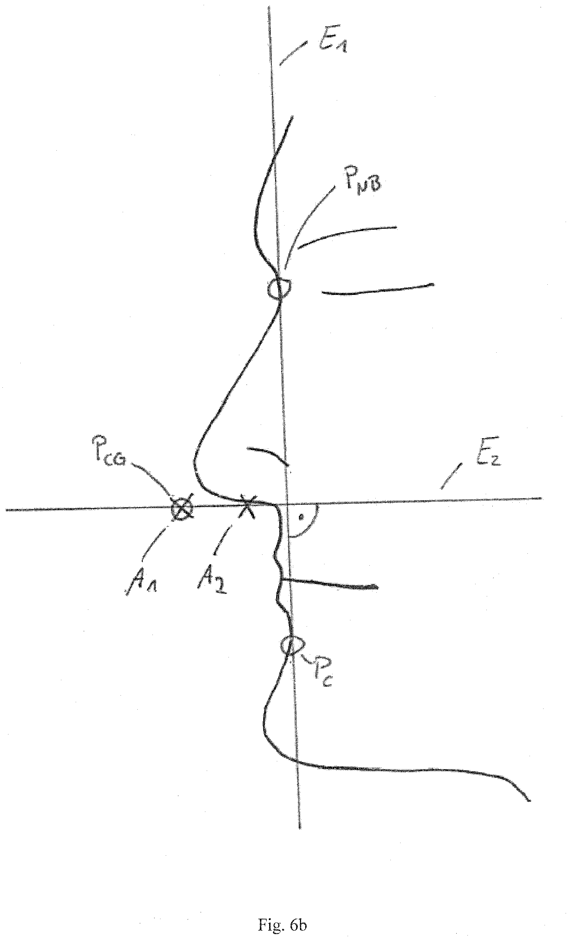

Fig. 6b is another side view indicating the axis of rotation and the frustrum.

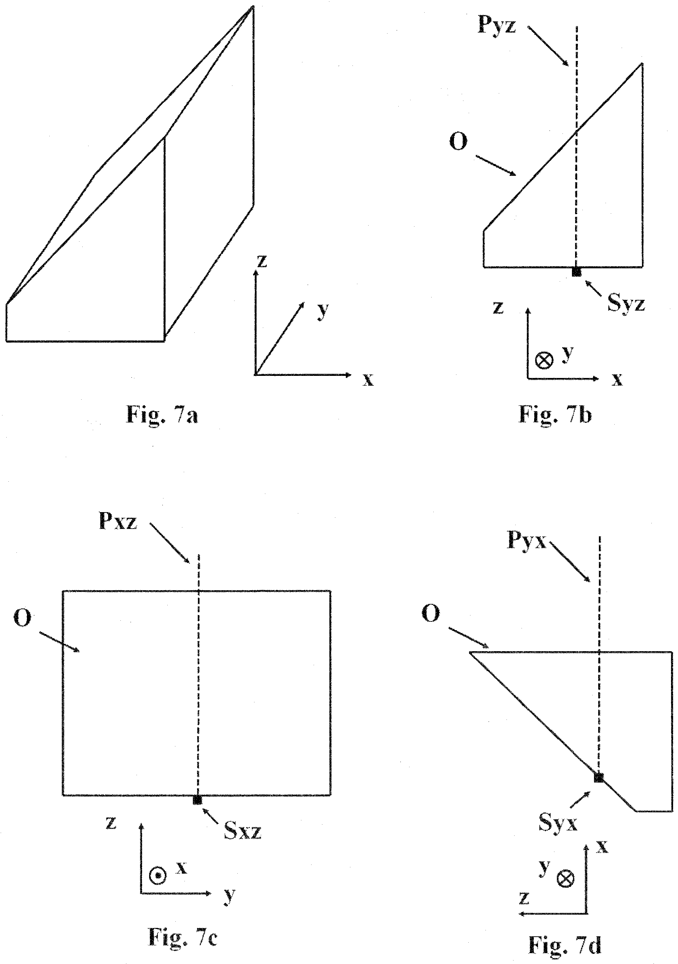

Figs. 7a to 7d illustrate a method to determine the center of mass of an object.

### 4 DETAILED DESCRIPTION OF EXAMPLES OF THE INVENTION

Before the invention is described in further detail, it is to be understood that the invention is not limited to the particular examples described herein, which may vary. It is also to be understood that the terminology used in this disclosure is for the purpose of describing only the particular examples discussed herein, and is not intended to be limiting.

The following description is provided in relation to various examples which may share one or more common characteristics and/or features. It is to be understood that one or more features of any one example may be combinable with one or more features of another example or other examples. In addition, any single feature or combination of features in any of the examples may constitute a further example.

### 4.1 THERAPY

In one form, the invention can be used for treating a respiratory disorder comprising the step of applying positive pressure to the entrance of the airways of a patient 1000.

In certain examples of the invention, a supply of air at positive pressure is provided to the nasal passages of the patient via one or both nares.

In certain examples of the invention, mouth breathing is limited, restricted or prevented.

### 4.2 TREATMENT SYSTEMS

In one form, the invention further comprises an apparatus or device for treating a respiratory disorder. The apparatus or device may comprise an RPT device 4000 for supplying pressurised air to the patient 1000 via an air circuit 4170 to a patient interface 3000.

### 4.3 PATIENT INTERFACE

A non-invasive patient interface 3000 in accordance with one aspect of the invention comprises the following functional aspects: a seal-forming structure 3100, a plenum chamber 3200. Further, a positioning and stabilising structure 3300 and one form of connection port for connection to air circuit 4170 may be provided. In some forms a functional aspect may be provided by one or more physical components. In some forms, one physical component may provide one or more functional aspects. In use the seal-forming structure 3100 may be arranged to surround an entrance to the airways of the patient so as to facilitate the supply of air at positive pressure to the airways.

Embodiments of the invention will now be described with reference to Figs. 4a to 4e and 5a to 5e. Herein, patient interfaces 3000 for delivering a supply of breathable gas to the patient's airways is depicted. The patient interface 3000 may be in the form of a full face mask, that is a mask adapted to cover both the mouth and the nose of a patient, or may be in the form of a nasal mask, that is a mask adapted to cover only the nose of a patient. Furthermore, other configurations of the patient interface are also possible. The patient interface comprises a seal-forming structure 3100 adapted to seal against a user's face. The seal-forming structure 3100 may be formed of resilient material adapted to conform to the patient's face. Furthermore, the patient interface comprises a shell 3250 being connected to the seal-forming structure 3100. The shell may be made of a relatively rigid material, such as polycarbonate to provide the patient interface 3000 with sufficient rigidity to withstand an increased pressure supplied to it. Furthermore, the patient interface 3000 comprises a frame 3800 adapted to be connected with a headgear 3300 (see Fig. 3). The frame, too, may be made of a rigid material to provide structural integrity. It may be made from the same material as the shell 3250. However, it is also possible to make the frame 3800 of another material than the shell 3250. In particular, the patient interface 3000 may be adapted such that under normal use, frame 3800 does not come into direct contact with a patient. In such a configuration, frame 3800 may be constructed from a non biocompatible (and therefore cheaper) material. Furthermore, the seal-forming structure 3100 and the shell 3250 form at least a part of a mask plenum chamber 3200 and the frame 3800 is coupled to the shell 3250 so as to allow a relative movement of the frame 3800 with respect to the shell 3250. This may provide an easy and convenient adjustability of the patient interface and allow the patient to suitably adjust the patient interface to his/her needs.

The frame 3800 may be adapted to be releasably coupled to the shell 3250. The shell 3250 may be directly coupled to the frame 3800, preferably by a form fit and/or a press fit.

The depicted patient interface 3000 further comprises a forehead support 3700 adapted to be positioned against the forehead of a patient; and the frame 3800 may be coupled to the forehead support 3700. To the forehead support 3700 may be attached at least one forehead support cushion adapted to contact the patient's forehead. Furthermore, the forehead support may be formed 3700 integrally with the frame 3800. The forehead support may provide a good support of the patient interface to the patient's face.

The shell 3250 may be indirectly coupled to the forehead support 3700 via the frame 3800.

The relative movement of the shell 3250 with respect to the frame 3800 may be a translatory and/or rotational movement. The relative movement of the shell 3250 with respect to the frame 3800 may be an adjustably lockable movement. In other words, a relative position of frame/forehead support to shell is adjustable and may be locked in the adjusted position. In particular, the lockability may be provided with discrete and distinct positions; that is, in only a certain number of predetermined positions, such as in only 2, 3, 4, 5, 6, 7, 8, 9 or 10 positions.

The frame 3800 may comprise a first 3830 and a second 3840 bar being laterally spaced apart, wherein each bar 3830, 3840 comprises one connection portion 3810, wherein the bars 3830, 3840 each comprise a first portion 3832, 3842, and wherein the first portions 3832, 3842 extend generally parallel to the longitudinal axis B-B of the patient interface (see, e.g. Fig. 5a).

The first portion may also be referred to as the upwardly extending portion or the upper portion of the bar. Furthermore, the longitudinal axis B-B is the axis of symmetry, that is an axis parallel to the plane of symmetry. More particularly, the patient interface 3000 may be of a substantially symmetrical shape to a plane of symmetry. This plane of symmetry is exemplarily depicted in Fig. 5a as B-B. However, as the Fig. is a 2 dimensional representation, the plane is depicted as a line or axis B-B of symmetry. When the term eyes of symmetry is used in this specification, what is meant is the axis B-B in a front view of the patient interface, as illustrated, e.g. in Fig. 5a.

The first and second bar 3830, 3840 may each comprise a second portion 3834, 3844, the second portions 3834, 3844 connecting the first portions 3832, 3842 with a bar 3710 of the forehead support 3700. Second portions 3834, 3844 and a bar 3710 of the forehead support may form a generally Y-shaped pattern. When viewed in an upright position, it may in particular be an inversely oriented Y-shaped pattern. The second portions 3834, 3844 may converge and join each other at a position located on or near the axis and/or plane of symmetry B-B of the patient interface 3000, and/or at or below the upper end of the shell 3250 and/or of the seal-forming structure 3100. The second portions 3834, 3844 may converge and, in use, join each other at a position in or adjacent the sagittal plane and/or in the region of the nose ridge or sellion. Preferably, this region comprises also slightly above or below the sellion, e.g. 2 cm above or below the sellion. The first and second bars 3830, 3840, particularly the first portions 3832, 3842 and/or the second portions 3834, 3844, may in particular not substantially protrude in the lateral direction x further than the respective outer edges or outer extents of the shell 3250 and/or of the seal-forming structure 3100. Such a patient interface may be less obtrusive than others.

The patient interface 3000 may further comprise means 3500 providing the relative movement, wherein the means is preferably located between a lower end 3252 and an upper end 3254 of the shell 3250 and/or of the seal forming structure 3100. The means will further be described with reference to Figs. 4a and 4e and Figs. 5a to 5e below.

In particular, the frame 3800 may comprises two connection portions 3810 each coupling with respective connection portions 3256 of the shell 3250 to form a pair 3256, 3810, and wherein the pairs are laterally spaced apart, laterally denoting a direction being perpendicular to the symmetry plane of the patient interface 3000. In the depicted embodiment, at least a portion of the shell 3250 and/or of the plenum chamber 3200, preferably the portion of the shell 3250 and/or of the plenum chamber 3200 receiving at least partially the nose, is/are located between the two pairs. Such a patient interface 3000 may in particular be less obstrusive. The patient interface less protruding from the wearer's face may further reduce the risk of unintentional displacement during the night when the patient moves.

A bar 3710 of the forehead support 3700 may be located in the sagittal plane and/or in the axis/plane of symmetry B-B, when in use.

The first and second bars 3830, 3840 may each comprise a third portion 3836, 3846 extending from the connecting portion 3810 downwardly. The third portion may also be referred to as the downwardly extending portion or lower portion of the bar. It may provide an easy grip and/or easy adjustment of the frame for better adaption to the face. The third portions 3836, 3846 may be connected via a connecting bar 3850 laterally connecting the first and second bar 3830, 3840, preferably at a lower end of the bars 3830, 3840. This feature may also help in providing an easy grip and increase rigidity. The third portions 3836, 3846 of the first and second bar 3830, 3840 may each comprise an attachment point for a strap of the headgear 3300. This may lead to better adaptability and a more rigid design since the connecting portion 3837 transmits the tension forces applied to the attachment point of one bar directly to the attachment point of the other bar. Furthermore, it may also reduces the risk of unintended disassembly.

The frame 3810 may comprise at least one locking means 3820 adjustably engaging with at least one locking means 3258 of the shell 3250. One of the locking means 3820, 3258 may be configured as a gear rod and/or gear rack, and the other of the locking means may be configured as a protrusion adapted to interengage the gear rod.

The frame 3810 may comprise at least one, preferably two locking means 3820 located adjacent the connecting portion 3810 of the first and/or second bars 3830, 3840.

The shell 3250 may comprise at least two side walls 3251 preferably being oriented substantially parallel to each other.

The side walls 3251 may extend substantially parallel to the first and/or second bar 3830, 3840.

The first and/or second bar 3830, 3840, preferably at their connecting portions 3810 in use may be arranged adjacent the side walls 3251.

The side wall 3251 may be configured as a recessed or offset portion of the shell 3250.

The side wall 3251 may be a planar wall portion.

The side wall 3251 may form a part of the walls limiting the plenum chamber 3200.

The side walls 3251 may be spaced apart in a lateral direction X, lateral being the direction perpendicular to the plane of symmetry.

In the embodiment depicted in Figs. 4a to 4e, the relative movement means are in the form of a slider 3500 having two protrusions 3810-2 being positioned in a groove 3256-2 of the shell 3250. The groove 3250 may be provided in a generally curved manner around a fulcrum having a respective radius. Thus, frame 3800 may "swing" around shell 3250. In other words, frame 3800 and shell 3250 may be supported swingable one to another.

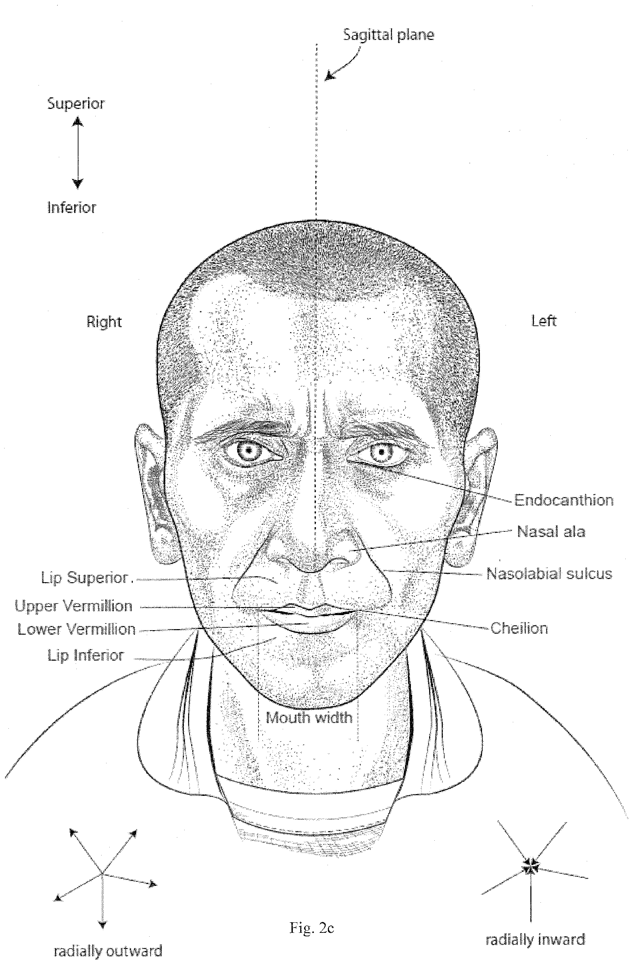

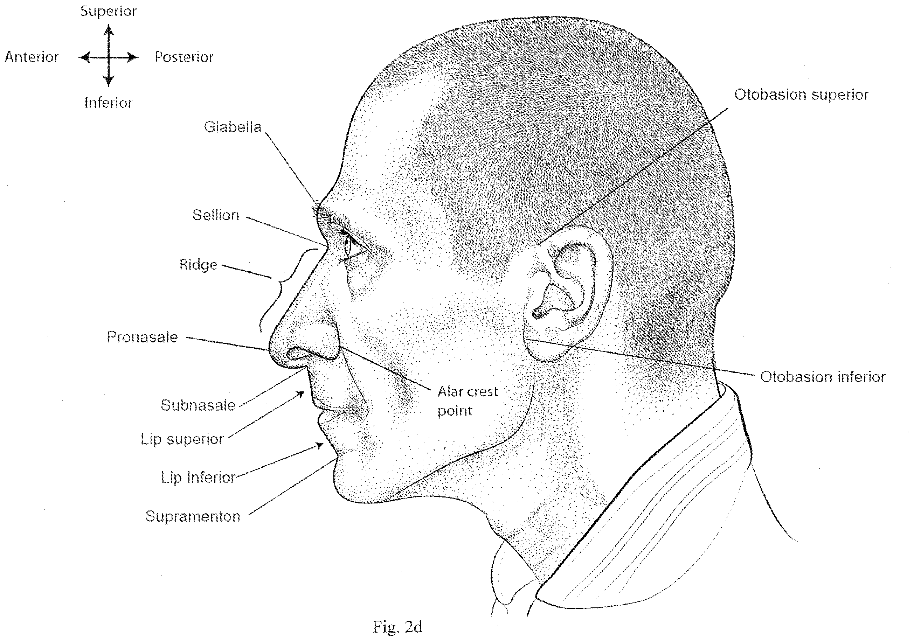

In this embodiment of the invention, the position of the fulcrum may be of some interest. This is most suitably defined in terms of its position on the patient's face when being worn and will now be described with further reference to Fig. 6b. In use, the seal forming structure 3100 contacts the patent inter alia at an uppermost sealing point located, in use, when the patient interface 3000 and the patient's head are in upright positions, at the uppermost end of the seal forming structure 3100. This uppermost end is preferably the uppermost end in the saggital plane of the patient. This uppermost point may further be the nasal bridge region and is denoted as the nasal bridge point P_{NB} in Fig. 6b. Furthermore, the sealing-forming structure 3100 also contacts the patient in use at a lowest sealing point, preferably again in the lowest point of the saggital plane. This point may be located on the chin and is therefore denoted as P_{C} in Fig. 6b. However, the person skilled in the art will understand that these points are merely exemplary, in particular for a full face mask covering both the patient's nose and mouth. If, however, another mask would be used, the exact locations of the uppermost and/or lowest sealing points could be different. If, e.g. a nasal mask was used as a patient interface, the lowest point would be located at the lip superior (see Figs. 2c and 2d). However, once the uppermost point P_{NB} and the lowest points P_{C} are defined, a frontal plane E1 is defined to include the uppermost sealing point P_{NB} and the lowest sealing point P_{C} and to be perpendicular to the saggital plane (which is parallel to the plane of the sheet of Fig. 6b) of the patient. Furthermore, a perpendicular plane E2 is defined to be perpendicular to the saggital plane and to the frontal plane E1 and to include the center of mass or center of gravity P_{CG} of a sub structure being composed of the seal forming structure 3100 and the shell 3250. For the sake of determining the center of mass of this sub structure, every portion formed integrally with the shell and/or the seal forming structure and every portion connecting these two should be construed to be a part of this sub structure. It is now preferred that the fulcrum A₂ (in Fig. 6b), A'-A' (in Fig. 6) is located close to (or within) the perpendicular plane E2, and preferably not more than 4 cm removed from that plane. Furthermore, a certain distance of approximately 0.5 cm to 10 cm or 1 cm to 8 cm, such as 3 cm to 6 cm, may be provided between the fulcrum A₂ and the center of mass P_{CG}.

In such a configuration the frame 3800 may swing around a fulcrum A₂, A'-A' provided close the plane E2, that is the plane also including the center of mass P_{CG}. In still other words, the means 3500, 3900 providing the relative movement may be located approximately in the middle of the distance between the lower end 3252 and the upper end 3254 of the shell 3250 and/or of the seal forming structure 3100 or the means may be provided approximately in the middle along the height of the mask when being worn in an upright position/wearing position. This also applies to the configuration depicted in Figs. 5a to 5e.

This may be a difference to prior art patient interfaces. By swinging around such a fulcrum being located approximately at the center of the height of the shell 3250 and/or seal-forming structure 3100, a suitable adjustment may be achieved. In particular, in such a configuration the uppermost and lowest portions of the seal forming structure 3100 may also be the portions of the seal forming structures 3100 being further distanced from the fulcrum and therefore undergoing the largest "swing" with respect to the frame 3800, thereby potentially allowing for better adjustability of the seal when compared to configurations where a fulcrum (or a rotational axis) is located close to one of the most extreme portions in the height direction of the seal forming structures.

Generally, the center of mass or center of gravity of an object, such as a patient interface or a sub structure thereof, may be determined as described with reference to Figs. 7a to 7d below. The object O depicted in Fig. 7a may be a regularly or irregularly shaped object. However, for sake of clarity and ease of understanding, a regularly shaped object O is used for this explanation. Its spatial coordinates may be described by variables x, y, z. One can determine the center of mass of such an object O by balancing the object O under the influence of gravity, by supporting the object O on a support Syz aligned with the y direction (see Fig. 7b) - in this notation, the first letter (here: y) indicates the direction of the support and the second letter (here: z) indicates the direction of the gravitational force. In the position of balance of Fig. 7b, there is an equal mass of the object O to the left and to the right of the center of mass plane Pyz in the y-z-plane. This plane is defined by the vector of the gravitational force (which is, in this orientation, the z-direction), the y direction and the support Syz; as Fig. 7b is a two dimensional representation with the center of mass plane Pyz being perpendicular to the plane of the drawing sheet in this representation, this center of mass plane Pyz is represented by a line. Thus, the y-z-center of mass plane Pyz is defined. Correspondingly, a x-z-center of mass plane Pxz is defined by balancing the object O on a support Sxz aligned with the x direction (see Fig. 7c). Finally, a y-x-center of mass plane Pyx is defined by turning the object O by 90° (around the y-axis) such that the x-direction of the object O becomes aligned to the direction of gravity and the object O is balanced, under the influence of gravity, with a support Syx aligned with the y direction (see Fig. 7d). The center of mass can now be determined as the intersection point of the three center of mass planes Pyz, Pxz and Pyx.

Another method to determine the center of mass of the patient interface or a sub-structure thereof would be the usage of plumb lines. In this method, one would suspend the structure on a thread (or the like) connected to the structure at a first location and let the structure hang freely. The structure will assume a position of balance, such that the plumb line (being in parallel to the thread) runs through the center of mass. Then, one would connect the thread at another location and repeat the procedure. Again, the plumb line will run though the center of mass of the structure. By determining the intersections of the plumb lines, one can determine the center of mass.

However, while these methods may be convenient ways to determine the center of mass, the skilled person may also employ other known methods to determine this center of mass.

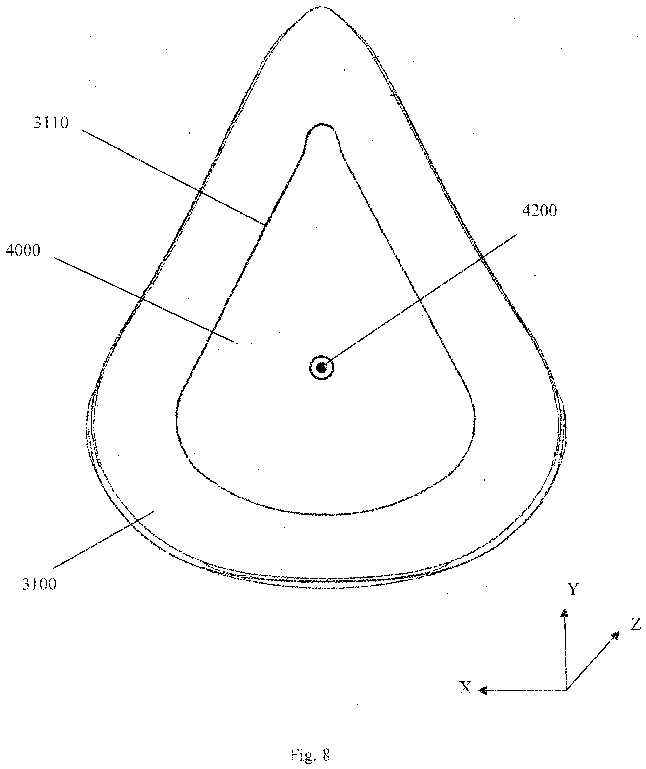

The position of the fulcrum A'-A' (in the embodiment depicted in Figs. 4a to 4e) or the rotation axis A-A (in the embodiment depicted in Figs. 5a to 5e) in the y-direction, i.e. the direction parallel to the vertical direction when the patient interface is worn and the patient is standing upright, may also be determined as follows. Reference is made to Fig. 8 depicting a seal forming structure 3100 when viewed from the patient side. Said seal forming structure 3100 defines an interior portion or interior area 4000, which is preferably delimited by an inner edge 3110 of the seal forming structure 3100. The interior portion 4000 is an opening providing the breathable gas to the patient. When pressurized gas is provided to the patient by means of the patient interface 3000, it is provided via the interior portion 4000. This creates a resultant force pushing the patient interface 3000 away from the patient. The resultant force results from the pressure being present at the interior portion 4000. The resultant force may be identified as a force vector 4200 located approximately at the centre of the interior portion 4000 (i.e. in such a way that there are equal areas or approximately equal areas above and below the force vector). That is, the location of the force vector 4200 coincides with the geometric centre of the interior area 4000. It is preferred that the position of the fulcrum A'-A' or the rotation axis A-A in the y-direction is close to the described geometric centre of the interior area 4000 or coincides therewith, in other words that the distance in the y-direction between the centre of the interior area 4100 of the seal forming structure 3100 and the fulcrum A'-A' or the rotation axis A-A, respectively, is less than 4 cm, preferably less than 2 cm, further preferably less than 1 cm, still more preferably less than 0,5 cm and most preferably less than 0,2 cm. Thus, the location of the fulcrum A'-A' or the rotation axis A-A in the y-direction would substantially coincide with the vector of the force resulting from the applied pressure. Put differently, the fulcrum A'-A' or the rotation axis A-A may be located at or near the "pneumatic centre point". Having the fulcrum A'-A' or the rotation axis A-A located in such a manner may be beneficial: The forces applied above and below the fulcrum A'-A' or the rotation axis A-A may therefore be approximately equal, helping to balance and locate the patient interface 3000 on the patient's face.

Such a configuration may also simplify finding the optimal fit of the patient interface 3000, e.g., eliminating or reducing leakage and/or reducing pressure points. Such fitting, e.g. the elimination of leakage and/or pressure points may be performed, when pressurised gas is supplied. Typically, in a first step, a headgear is connected to headgear connectors of the patient interface (see, e.g., the patient interface in Fig. 7 of WO 98/04310 A1). Then, while pressurized gas is supplied to the patient interface, the headgear tension needs to be adjusted in order to eliminate leakage and achieve a desired level of comfort for the wearer. Finding the ideal configuration of the patient interface may be difficult: After the headgear is attached and the patient interface is pressurized, the patient interface tends to tilt away from the patient's face, as the resultant force applied by the pressure is above the location of where the patient interface is supported (by lower headgear connectors) against the wearer's face, which for full face masks may typically be in an area near the side of the mouth. In turn, tilting the patient interface towards the patient is more difficult (i.e. one has to overcome a higher force) than tilting the patient interface away from the patient. This may complicate the finding of the best fit. This is overcome by the present configuration: The patient interfaces 3000 of the present configuration may be applied to the patient's face and headgear may be secured to the patient interface 3000 on lower headgear connectors (also referred to as attachment points for straps). The patient interface may then be pressurized. By means of the frame 3800, the holding force created by headgear attached to lower headgear connectors in the frame 3800 acts on the remainder of the patient interface 3000 via the connection portion 3810 of the frame. Thus, there is no substantial offset in the y-direction between the resultant force from the applied pressurized gas and the connection between the headgear and the patient interface 3000, such that the patient interface 3000 is not tilted (or at least: is not tilted with the same force) as in the prior art arrangement. Tilting an upper portion of the patient interface 3000 towards and away from the patient may therefore require similar forces, simplifying correct adjustment of the patient interface 3000.

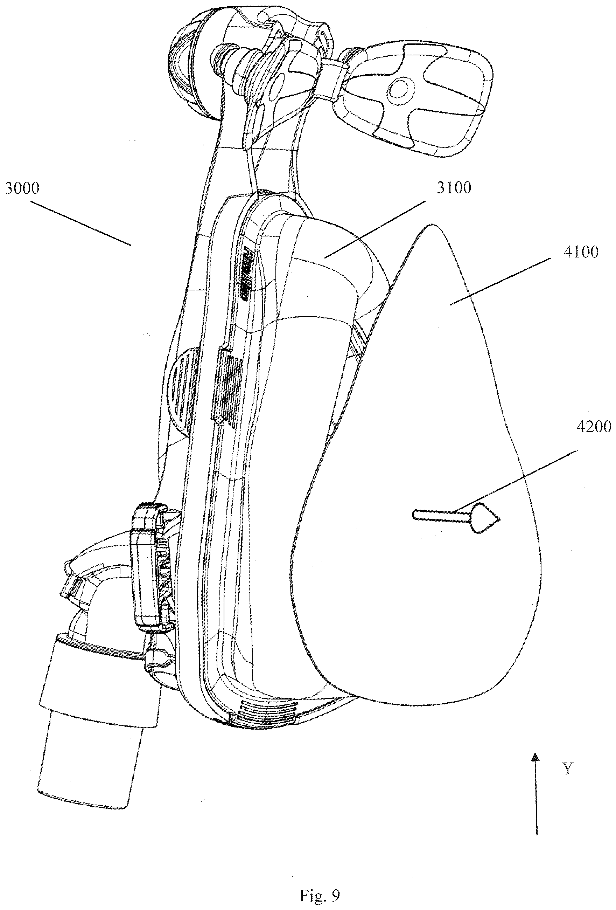

In some embodiments, the seal forming structure 3100 may comprise a membrane to come into contact with the patient's face (see, e.g. the cross sectional view of Figures 4c and 5c). In such embodiments, a pressure created in the patient interface 3000 may also press the patient interface 3000 against the patient's face, when headgear is attached to the patient interface 3000. That is, in such embodiments also the portion of the seal forming structure 3100 may push the patient interface 300 away from the patient's head, which when headgear holds the patient interface 3000 in place, creates a sealing force. Therefore, in some embodiments, it may be more appropriate not to only consider the interior portion of patient interface 3000 to constitute the portion pushing the patient interface 3000 away from the patient, but in fact the complete area of the mask plenum chamber 3200 when viewed from the patient - in Figure 8, this would be the combination of the interior area 4000 and the seal forming structure 3100. The resultant force vector may then be located at the geometric centre of the projected area of the complete mask plenum chamber as defined, e.g. by the outside border of the seal forming structure 3100 - this is also illustrated in Fig. 9 showing an exemplary patient interface 3000 having a seal forming structure 3100 and projected area 4100 of the mask plenum chamber. In the geometric centre of this projected area 4100, there is located the resultant force vector 4200. In line with the above rationale, it may be advantageous to locate the fulcrum A'-A' or the rotation axis A-A close to the geometric centre of mask plenum chamber in the y-direction, e.g. such that the two are displaced in the y-direction by less than 4 cm from one another, preferably less than 2 cm, further preferably less than 1 cm, still more preferably less than 0,5 cm and most preferably less than 0,2 cm.

In some embodiments, the locking means 3820 of the frame 3800 may be configured as a gear rod 3820-2 and the locking means 3258 of the shell 3250 may comprise the protrusion 3258-2 engaging the gear rods 3820-2.

Each protrusion 3258-2 may located at a bendable web portion 3258-3, the web portion 3258-3 being adapted to move the protrusion from an interlocked position to an non-interlocked position. Thereby adjustment of the frame 3800 relative to the shell 3250 may be allowed. The bendable web portions is/are integrally formed with the shell 3250. In particular, the protrusions 3258-2 may laterally engage the gear rods 3820-2.

The at least one, preferably two bendable web portions 3258-3 may be arranged adjacent and generally parallel to the first and/or second bars 3830, 3840, the bendable web portions being adapted to bend towards the axis and/or plane of symmetry B-B. Further, the protrusion 3258-2 of the bendable web portion 3258-3 may generally extend in the lateral direction X.

The shell 3250 may comprise two bendable web portions 3258-2 adapted to be operated by two fingers.

The gear rod may extend from the outer surface of the first and/or second bars 3830, 3840.

The gear rod may have or may be located on a generally curved or acuate outer surface.

The connection portion 3256 of the shell 3250 may be configured as a groove 3256-2, preferably extending in the lateral direction X of the patient interface 3000.

The groove may also be referred to as or keyway or notch.

The means to allow the relative movement may be configured as a sliding arrangement 3500.

The sliding arrangement 3500 may be configured such that the frame 3800 slides relative to the shell 3250 along a curved path with a fulcrum A'-A'.

The fulcrum may be referred to as a rotation axis wherein the rotation axis is displaced from the entity adapted to move rotationally. The curved path may in particular be a circular or elliptical arc.

The shell 3250 and the frame 3800 may be coupled via the at least one groove 3256-2 of the shell 3250 and the at least one protrusion 3810-2 of the frame preferably extending in the lateral direction X of the patient interface 3000.

This may allow a relative movement of the shell 3250 with respect to the frame 3800.

The at least one groove 3256-2 may have a generally curved path, preferably so that the frame 3800 follows a generally curved path.

Two grooves 3256-2 having a curved path may be located on two side walls 3251 of the shell 3250, each groove 3256-2 being engaged by at least one, preferably two laterally extending protrusions 3810-2 of the first and second bars 3830, 3840 located adjacent the side walls 3251, wherein the protrusions 3810-2 are adapted to slide inside the grooves 3256-2, preferably such that the frame 3800 follows a generally curved path.

One laterally extending protrusion 3810-2 may engage each groove 3256-2, the laterally extending protrusion 3810-2 having a curved shape that correspond to the curvature of the groove.

A plurality (such as two) of laterally extending protrusion 3810-2 may engage each groove 3256-2, the plurality of laterally extending protrusion 3810-2 having a generally round shape and being located on a curved path corresponding to the curved path of the groove 3256-2.

In particular, corresponding curved paths may have substantially the same fulcrum.

In use, the fulcrum A'-A' of the sliding arrangement 3500 may be located in an [outwardly oriented] offset distance from the face of the patient of about - 2 cm to about 8 cm, preferably of about 0 cm to about 3 cm, and most preferably of about 1,5 cm.

That is, -2 cm is a negative offset; i.e. fulcrum located at a distance of 2 cm oriented inwardly.

With particular reference to the embodiment depicted in Figs. 5a to 5e, it is noted that the locking means 3820 of the frame 3800 may be configured as a protrusion 3820-1, and the locking means 3258 of the shell 3250 may be configured as a gear rod 3258-1.

The locking means 3820 of the frame 3800 may located at the connecting bar 3850.

The locking means 3820 located at the connecting bar 3850 may be configured as a protrusion 3820-1, and the connecting bar 3850 and the first and second bars 3830, 3840, preferably the third portions 3836, 3846 thereof, may be adapted to flexibly bend, preferably so as to disengage the protrusion 3820-1 when the third portions 3836, 3846 are pressed laterally towards the axis/plane of symmetry B-B. Thereby, adjustment of the position of the frame 3400 relative to the shell 3250 may be allowed. The disengagement of the protrusion may in particular be a disengagement from the gear rod. In other word, the third portion is pressed laterally towards the axis/plane of symmetry B-B or pressed laterally inwardly, i.e. towards each other on this configuration.

In particular, the third portions 3836, 3846 may be pressed laterally towards the axis/plane of symmetry B-B at predetermined, preferably surface increased, push areas 3837, 3847.

The shell 3250 may comprise at least one, preferably two shoulders 3259 extending generally parallel to the third portions 3836, 3846, wherein the shoulders 3259 are located between the push areas 3837, 3847 and the connecting portions 3810; and wherein portions 3836-1, 3846-1 of the third portions 3836, 3846 adjacent the shoulders 3259 are adapted to abut against the shoulders when third portions are pressed laterally towards the axis of symmetry B-B.

The connection portion 3256 of the shell 3250 may be configured as a hole 3256-1 preferably extending in the lateral direction X of the patient interface 3000.

If located in the side wall, the hole 3256-1 may be an indentation or round recess.

The means to allow the relative movement may be configured as a joint 3900 adapted to rotate around an rotation axis A-A.

The joint may also be referred to as a hinge, a pivot, or an articulated joint.

The shell 3250 and the frame 3800 may be coupled via the at least one hole 3256-1 of the shell 3250 and at least one protrusion 3810-1 of the frame preferably extending in the lateral direction X of the patient interface 3000.

This may allow a relative movement of the shell 3250 with respect to the frame 3800.

Two holes 3256-1 may be located in two protruding web portions 3253 of the shell 3250, each hole 3256-1 being engaged by one laterally extending protrusion 3810-1 of the first and second bars 3830, 3840 located adjacent the protruding web portions 3253, wherein each protrusion 3810-2 is adapted to rotate inside the hole 3256-1.

The longitudinal axis B-B may be the axis of symmetry or is included in the plane of symmetry.

The rotation axis A-A may be located in the application position outwardly offset from plenum chamber 3200.

In other words, the rotation axis is offset in a direction Z away from the patient indicated in Fig. 2e. I.e. in a direction generally orthogonal to longitudinal and lateral directions X, Y.

In use, the rotation axis A-A of the joint 3900 may be located in an offset distance from the face of the patient of about 2 cm to about 10 cm, preferably of about 3 cm to about 6 cm, and most preferably of about 4,5 cm.

The offset is in particular in the direction Z away from the patient's face.

In this embodiment, the position of the rotation axis may be of some interest. This is most suitably defined in terms of its position on the patient's face when being worn and will also be described with further reference to Fig. 6b. In use, the seal forming structure 3100 contacts the patent inter alia at an uppermost sealing point located, in use, when the patient interface 3000 and the patient's head are in upright positions, at the uppermost end of the seal forming structure 3100. This uppermost end is preferably the uppermost end in the saggital plane of the patient. This uppermost point may further be the nasal bridge region and is denoted as the nasal bridge point P_{NB} in Fig. 6b. Furthermore, the sealing-forming structure 3100 also contacts the patient in use at a lowest sealing point, preferably again in the lowest point of the saggital plane. This point may be located on the chin and is therefore denoted as P_{C} in Fig. 6b. However, the person skilled in the art will understand that these points are merely exemplary, in particular for a full face mask covering both the patient's nose and mouth. If, however, another mask would be used, the exact locations of the uppermost and/or lowest sealing points could be different. If, e.g. a nasal mask was used as a patient interface, the lowest point would be located at the lip superior (see Figs. 2c and 2d). However, once the uppermost point P_{NB} and the lowest points P_{C} are defined, a frontal plane E1 is defined to include the uppermost sealing point P_{NB} and the lowest sealing point P_{C} and to be perpendicular to the saggital plane (which is parallel to the plane of the sheet of Fig. 6b) of the patient. Furthermore, a perpendicular plane E2 is defined to be perpendicular to the saggital plane and to the frontal plane E1 and to include the center of mass or center of gravity P_{CG} of a sub structure being composed of the seal forming structure 3100 and the shell 3250. It is now preferred that the rotation axis A₁ (in Fig. 6b), A-A (in Fig. 6) is located close to (or within) the perpendicular plane E2, and preferably not more than 4 cm removed from that plane. Furthermore, it is also preferred that it is located overall close to the center of mass P_{CG}. In this regard, it is also noted that the terms center of mass and center of gravity are used interchangeable in this specification.

With reference again to both embodiments, it is noted that the groove 3256-2 and/or the hole 3256-1 may be located in the side wall 3251.

The shell 3250 may comprise at least one, preferably two protruding web portions 3253, wherein the holes 3256-1 and/or grooves 3256-2 is/are located at the protruding web portions 3253.

The protruding web portions 3253 may generally protrude in a direction Z perpendicular to the lateral and longitudinal directions X,Y.

In other words, the portions extend in use in a direction away from the patient's face.

The groove 3256-2 and/or the hole 3256-1 may be adapted to be engaged by the connection portion 3810 of the frame 3800.

At least one, preferably all connection portions 3810 of the frame 3800 may be configured as a protrusion 3810-1 or a plurality of protrusions 3810-2 preferably extending in the lateral direction X of the patient interface 3000.

The groove 3256-2 and/or the hole 3256-1 and the protrusions 3810-1, 3810-2 may be adapted to engage and/or disengage upon bending of the frame 3800 in the lateral direction of the patient interface 3000.

The frame 3800, preferably the first and second bars 3830, 3840, and most preferably the first and/or second portions 3832, 3842; 3834, 3844 of the first and second bars 3830, 3840 may be adapted to bend, preferably when a lateral force F is applied to the first and/or second portions 3832, 3842; 3834, 3844.

The groove 3256-2 and/or the hole 3256-1 may be located in the side wall 3251, and the protrusions 3810-1, 3810-2 may be adapted to engage and/or disengage the groove 3256-2 and/or the hole 3256-1 when the first and/or second portions 3832, 3842; 3834, 3844 are pressed in a lateral direction X away from each other, preferably by applying a laterally outwardly oriented force F to grips 3833, 3843 preferably located at the first portions 3832, 3842.

The groove 3256-2 and/or the hole 3256-1 may be located in the protruding web portions 3253, and the protrusions 3810-1, 3810-2 may be adapted to engage and/or disengage the groove 3256-2 and/or the hole 3256-1 when the first and/or second portions 3832, 3842; 3834, 3844 are pressed in a lateral direction X towards each other, preferably by applying a laterally inwardly oriented force F to grips 3833, 3843 preferably located at the first portions 3832, 3842.

The rotation axis A-A of the joint 3900 and/or the fulcrum A'-A' of the sliding arrangement 3500 may extend perpendicular to the longitudinal axis B-B of the patient interface 3000 in a lateral direction X.

The rotation axis A-A of the joint 3900 and/or the fulcrum A'-A' of the sliding arrangement 3500 may be located between an lower end 3252 and an upper end 3254 of the shell 3250 and/or of the seal-forming structure 3100. 7

Upper and lower ends 3252, 3254 refer to the upper and lower ends of the shell 3250 in use; i.e. in the longitudinal direction Y.

In use, the rotation axis A-A of the joint 3900 and/or the fulcrum A'-A' of the sliding arrangement 3500 may be located in a vertical direction Y between the sellion and the mouth of a patient, preferably at the nasal ridge, pronasale or lip superior.

The rotation axis A-A and/or the fulcrum A'-A' of the sliding arrangement 3500 may extend through the plenum chamber 3200, the shell 3250 and/or the seal-forming structure 3100.

The rotation axis A-A of the joint 3900 and/or the fulcrum A'-A' of the sliding arrangement 3500 may be located in or adjacent the center of gravity of the shell 3250 with seal-forming structure 3100, of the frame 3800, the patient interface 3000 and/or the mask.

In other words, the pivoting point or hinge is positioned at a centre of gravity or alternatively the centroid of the mask. This may allow for adjustment without having an impact on the seal. Adjacent may mean that the respective two structures are displaced by no more than 4 cm, 3 cm, 2 cm, 1 cm or 0,5 cm from one another.

The patient interface 3000 may be adapted for delivering breathable gas at positive pressure, preferably for a PAP treatment.

The patient interface 3000 may comprise a means for adjustably adapting the relative position of the frame 3800 to the forehead support 3700.

### 4.3.1 Seal-forming structure

In one form of the invention, a seal-forming structure 3100 provides a seal-forming surface, and may additionally provide a cushioning function.

A seal-forming structure 3100 in accordance with the invention may be constructed from a soft, flexible, resilient material such as silicone.

In one form, the seal-forming structure 3100 comprises a sealing flange and a support flange. The sealing flange 3110 comprises a relatively thin member with a thickness of less than about 1mm, for example about 0.25mm to about 0.45mm, that extends around the perimeter of the plenum chamber 3200. Support flange 3120 may be relatively thicker than the sealing flange 3110. The support flange 3120 is disposed between the sealing flange 3110 and the marginal edge of the plenum chamber 3200, and extends at least part of the way around the perimeter 3210. The support flange 3120 is or includes a spring-like element and functions to support the sealing flange 3110 from buckling in use. In use the sealing flange 3110 can readily respond to system pressure in the plenum chamber 3200 acting on its underside to urge it into tight sealing engagement with the face.

In one form, the non-invasive patient interface 3000 comprises a seal-forming portion that forms a seal in use on an upper lip region (that is, the *lip superior*) of the patient's face.

In one form the non-invasive patient interface 3000 comprises a seal-forming portion that forms a seal in use on a chin-region of the patient's face.

### 4.3.2 Plenum chamber

The plenum chamber 3200 has a perimeter 3210 that is shaped to be complementary to the surface contour of the face of an average person in the region where a seal will form in use. In use, a marginal edge 3220 of the plenum chamber 3200 is positioned in close proximity to an adjacent surface of the face. Actual contact with the face is provided by the seal-forming structure 3100. The seal-forming structure 3100 may extend in use about the entire perimeter 3210 of the plenum chamber 3200.

### 4.3.3 Positioning and stabilising structure 3300

The seal-forming portion 3100 of the patient interface 3000 of the invention may be held in sealing position in use by the positioning and stabilising structure 3300.

In one form of the invention, a positioning and stabilising structure 3300 is provided that is configured in a manner consistent with being worn by a patient while sleeping. In one example the positioning and stabilising structure 3300 has a low profile, or cross-sectional thickness, to reduce the perceived or actual bulk of the apparatus. In one example, the positioning and stabilising structure 3300 comprises at least one strap having a rectangular cross-section. In one example the positioning and stabilising structure 3300 comprises at least one flat strap.

In one form of the invention, a positioning and stabilising structure 3300 comprises a strap constructed from a laminate of a fabric patient-contacting layer, a foam inner layer and a fabric outer layer. In one form, the foam is porous to allow moisture, (e.g., sweat), to pass through the strap. In one form, the fabric outer layer comprises loop material to engage with a hook material portion.

In certain forms of the invention, a positioning and stabilising structure 3300 comprises a strap that is extensible, e.g. resiliently extensible. For example the strap may be configured in use to be in tension, and to direct a force to draw a cushion into sealing contact with a portion of a patient's face. In an example the strap may be configured as a tie.

In certain forms of the invention, a positioning and stabilising structure 3300 comprises a strap that is bendable and e.g. non-rigid. An advantage of this aspect is that the strap is more comfortable for a patient to lie upon while the patient is sleeping.

### 4.3.4 Vent

In one form, the patient interface 3000 includes a vent 3400 constructed and arranged to allow for the washout of exhaled carbon dioxide.

One form of vent 3400 in accordance with the invention comprises a plurality of holes, for example, about 20 to about 80 holes, or about 40 to about 60 holes, or about 45 to about 55 holes.

The vent 3400 may be located in the plenum chamber 3200. Alternatively, the vent 3400 is located in a decoupling structure, e.g., a swivel.

### 4.3.5 Decoupling structure(s)

In one form the patient interface 3000 includes at least one decoupling structure 3500, for example, a swivel 3510 or a ball and socket 3520.

### 4.3.6 Connection port

Connection port allows for connection to the air circuit 4170.

### 4.3.7 Forehead support

In one form, the patient interface 3000 includes a forehead support 3700.

### 4.3.8 Anti-asphyxia valve

In one form, the patient interface 3000 includes an anti-asphyxia valve.

### 4.3.9 Ports

In one form of the invention, a patient interface 3000 includes one or more ports that allow access to the volume within the plenum chamber 3200. In one form this allows a clinician to supply supplemental oxygen. In one form, this allows for the direct measurement of a property of gases within the plenum chamber 3200, such as the pressure.

### 4.3.10 Frame 3800

In one form, the patient interface 300 includes a frame 3800 that bears the load of tension between two or more connections with a headgear.

### 4.4 GLOSSARY

For the purposes of the invention disclosure, in certain forms of the invention, one or more of the following definitions may apply. In other forms of the invention, alternative definitions may apply.

### 4.4.1 General

*Air*: In certain forms of the invention, air may be taken to mean atmospheric air, and in other forms of the invention air may be taken to mean some other combination of breathable gases, e.g. atmospheric air enriched with oxygen.

*Ambient*: In certain forms of the invention, the term ambient will be taken to mean (i) external of the treatment system or patient, and (ii) immediately surrounding the treatment system or patient.

For example, ambient humidity with respect to a humidifier may be the humidity of air immediately surrounding the humidifier, e.g. the humidity in the room where a patient is sleeping. Such ambient humidity may be different to the humidity outside the room where a patient is sleeping.

In another example, ambient pressure may be the pressure immediately surrounding or external to the body.

In certain forms, ambient (e.g., acoustic) noise may be considered to be the background noise level in the room where a patient is located, other than for example, noise generated by an RPT device or emanating from a mask or patient interface. Ambient noise may be generated by sources outside the room.

*Continuous Positive Airway Pressure (CPAP) therapy*: CPAP therapy will be taken to mean the application of a supply of air to an entrance to the airways at a pressure that is continuously positive with respect to atmosphere. The pressure may be approximately constant through a respiratory cycle of a patient. In some forms, the pressure at the entrance to the airways will be slightly higher during exhalation, and slightly lower during inhalation. In some forms, the pressure will vary between different respiratory cycles of the patient, for example, being increased in response to detection of indications of partial upper airway obstruction, and decreased in the absence of indications of partial upper airway obstruction.

*Patient*: A person, whether or not they are suffering from a respiratory disease.

*Automatic Positive Airway Pressure (APAP) therapy*: CPAP therapy in which the treatment pressure is automatically adjustable, e.g. from breath to breath, between minimum and maximum limits, depending on the presence or absence of indications of SDB events.

### 4.4.2 Anatomy of the face

*Ala:* the external outer wall or "wing" of each nostril (plural: alar)

*Alare:* The most lateral point on the nasal *ala.*

*Alar curvature (or alar crest) point:* The most posterior point in the curved base line of each *ala,* found in the crease formed by the union of the *ala* with the cheek.

*Auricle:* The whole external visible part of the ear.

*(nose) Bony framework:* The bony framework of the nose comprises the nasal bones, the frontal process of the maxillae and the nasal part of the frontal bone.

*(nose) Cartilaginous framework:* The cartilaginous framework of the nose comprises the septal, lateral, major and minor cartilages.

*Columella:* the strip of skin that separates the nares and which runs from the *pronasale* to the upper lip.

*Columella angle:* The angle between the line drawn through the midpoint of the nostril aperture and a line drawn perpendicular to the Frankfurt horizontal while intersecting subnasale.

*Frankfort horizontal plane*: A line extending from the most inferior point of the orbital margin to the left tragion. The tragion is the deepest point in the notch superior to the tragus of the auricle.

*Glabella:* Located on the soft tissue, the most prominent point in the midsagittal plane of the forehead.

*Lateral nasal cartilage:* A generally triangular plate of cartilage. Its superior margin is attached to the nasal bone and frontal process of the maxilla, and its inferior margin is connected to the greater alar cartilage.

*Lip, lower (labrale inferius):*
*Lip, upper (labrale superius):*
*Greater alar cartilage:* A plate of cartilage lying below the lateral nasal cartilage. It is curved around the anterior part of the naris. Its posterior end is connected to the frontal process of the maxilla by a tough fibrous membrane containing three or four minor cartilages of the *ala.*

*Nares (Nostrils)*: Approximately ellipsoidal apertures forming the entrance to the nasal cavity. The singular form of nares is naris (nostril). The nares are separated by the nasal septum.

*Naso-labial sulcus or Naso-labial fold*: The skin fold or groove that runs from each side of the nose to the corners of the mouth, separating the cheeks from the upper lip.

*Naso-labial angle*: The angle between the columella and the upper lip, while intersecting subnasale.

*Otobasion inferior*: The lowest point of attachment of the auricle to the skin of the face.

*Otobasion superior*: The highest point of attachment of the auricle to the skin of the face.

*Pronasale*: the most protruded point or tip of the nose, which can be identified in lateral view of the rest of the portion of the head.

*Philtrum*: the midline groove that runs from lower border of the nasal septum to the top of the lip in the upper lip region.

*Pogonion*: Located on the soft tissue, the most anterior midpoint of the chin.

*Ridge (nasal)*: The nasal ridge is the midline prominence of the nose, extending from the Sellion to the Pronasale.

*Sagittal plane*: A vertical plane that passes from anterior (front) to posterior (rear) dividing the body into right and left halves.

*Sellion:* Located on the soft tissue, the most concave point overlying the area of the frontonasal suture.

*Septal cartilage (nasal)*: The nasal septal cartilage forms part of the septum and divides the front part of the nasal cavity.

*Subalare*: The point at the lower margin of the alar base, where the alar base joins with the skin of the superior (upper) lip.

*Subnasal point*: Located on the soft tissue, the point at which the columella merges with the upper lip in the midsagittal plane.

*Supramentale*: The point of greatest concavity in the midline of the lower lip between labrale inferius and soft tissue pogonion

### 4.4.3 Anatomy of the skull

*Frontal bone*: The frontal bone includes a large vertical portion, the *squama frontalis,* corresponding to the region known as the forehead.

*Mandible*: The mandible forms the lower jaw. The mental protuberance is the bony protuberance of the jaw that forms the chin.

*Maxilla*: The maxilla forms the upper jaw and is located above the mandible and below the orbits. The *frontal process of the maxilla* projects upwards by the side of the nose, and forms part of its lateral boundary.

*Nasal bones*: The nasal bones are two small oblong bones, varying in size and form in different individuals; they are placed side by side at the middle and upper part of the face, and form, by their junction, the "bridge" of the nose.

*Nasion:* The intersection of the frontal bone and the two nasal bones, a depressed area directly between the eyes and superior to the bridge of the nose.

*Occipital bone*: The occipital bone is situated at the back and lower part of the cranium. It includes an oval aperture, the *foramen magnum,* through which the cranial cavity communicates with the vertebral canal. The curved plate behind the foramen magnum is the *squama occipitalis.*

*Orbit*: The bony cavity in the skull to contain the eyeball.

*Parietal bones*: The parietal bones are the bones that, when joined together, form the roof and sides of the cranium.

*Temporal bones*: The temporal bones are situated on the bases and sides of the skull, and support that part of the face known as the temple.

*Zygomatic bones*: The face includes two zygomatic bones, located in the upper and lateral parts of the face and forming the prominence of the cheek.

### 4.4.4 Anatomy of the respiratory system

*Diaphragm:* A sheet of muscle that extends across the bottom of the rib cage. The diaphragm separates the thoracic cavity, containing the heart, lungs and ribs, from the abdominal cavity. As the diaphragm contracts the volume of the thoracic cavity increases and air is drawn into the lungs.

*Larynx*: The larynx, or voice box houses the vocal folds and connects the inferior part of the pharynx (hypopharynx) with the trachea.

*Lungs*: The organs of respiration in humans. The conducting zone of the lungs contains the trachea, the bronchi, the bronchioles, and the terminal bronchioles. The respiratory zone contains the respiratory bronchioles, the alveolar ducts, and the alveoli.

*Nasal cavity*: The nasal cavity (or nasal fossa) is a large air filled space above and behind the nose in the middle of the face. The nasal cavity is divided in two by a vertical fin called the nasal septum. On the sides of the nasal cavity are three horizontal outgrowths called nasal conchae (singular "concha") or turbinates. To the front of the nasal cavity is the nose, while the back blends, via the choanae, into the nasopharynx.

*Pharynx:* The part of the throat situated immediately inferior to (below) the nasal cavity, and superior to the oesophagus and larynx. The pharynx is conventionally divided into three sections: the nasopharynx (epipharynx) (the nasal part of the pharynx), the oropharynx (mesopharynx) (the oral part of the pharynx), and the laryngopharynx (hypopharynx).

### 4.4.5 Materials

*Silicone or Silicone Elastomer*: A synthetic rubber. In this specification, a reference to silicone is a reference to liquid silicone rubber (LSR) or a compression moulded silicone rubber (CMSR). One form of commercially available LSR is SILASTIC (included in the range of products sold under this trademark), manufactured by Dow Corning. Another manufacturer of LSR is Wacker. Unless otherwise specified to the contrary, an exemplary form of LSR has a Shore A (or Type A) indentation hardness in the range of about 35 to about 45 as measured using ASTM D2240

*Polycarbonate*: a typically transparent thermoplastic polymer of Bisphenol-A Carbonate.

### 4.4.6 Aspects of a patient interface

*Anti-asphyxia valve (AAV):* The component or sub-assembly of a mask system that, by opening to atmosphere in a failsafe manner, reduces the risk of excessive CO₂ rebreathing by a patient.

*Elbow:* A conduit that directs an axis of flow of air to change direction through an angle. In one form, the angle may be approximately 90 degrees. In another form, the angle may be less than 90 degrees. The conduit may have an approximately circular cross-section. In another form the conduit may have an oval or a rectangular cross-section.

*Frame*: Frame will be taken to mean a mask structure that bears the load of tension between two or more points of connection with a headgear. A mask frame may be a non-airtight load bearing structure in the mask. However, some forms of mask frame may also be air-tight.

*Headgear:* Headgear will be taken to mean a form of positioning and stabilizing structure designed for use on a head. Preferably the headgear comprises a collection of one or more struts, ties and stiffeners configured to locate and retain a patient interface in position on a patient's face for delivery of respiratory therapy. Some ties are formed of a soft, flexible, elastic material such as a laminated composite of foam and fabric.

*Membrane*: Membrane will be taken to mean a typically thin element that has, preferably, substantially no resistance to bending, but has resistance to being stretched.

*Plenum chamber*: a mask plenum chamber will be taken to mean a portion of a patient interface having walls enclosing a volume of space, the volume having air therein pressurised above atmospheric pressure in use. A shell may form part of the walls of a mask plenum chamber.

*Seal*: The noun form ("a seal") will be taken to mean a structure or barrier that intentionally resists the flow of air through the interface of two surfaces. The verb form ("to seal") will be taken to mean to resist a flow of air.

*Shell*: A shell will be taken to mean a curved, relatively thin structure preferably having bending, tensile and compressive stiffness. For example, a curved structural wall of a mask may be a shell. The shell may be configured to be rigid, semi-rigid or soft. In some forms, a shell may be faceted. In some forms a shell may be airtight. In some forms a shell may not be airtight.

*Stiffener*: A stiffener will be taken to mean a structural component designed to increase the bending resistance of another component in at least one direction.

*Strut:* A strut will be taken to be a structural component designed to increase the compression resistance of another component in at least one direction.

*Swivel*: (noun) A subassembly of components configured to rotate about a common axis, preferably independently, preferably under low torque. In one form, the swivel may be constructed to rotate through an angle of at least 360 degrees. In another form, the swivel may be constructed to rotate through an angle less than 360 degrees. When used in the context of an air delivery conduit, the sub-assembly of components preferably comprises a matched pair of cylindrical conduits. There may be little or no leak flow of air from the swivel in use.

*Tie*: A tie will be taken to be a structural component designed to resist tension.

*Vent*: (noun) the structure that allows an intentional flow of air from an interior of the mask, or conduit to ambient air, e.g. to allow washout of exhaled gases.

### 4.4.7 Terms used in relation to patient interface

*Curvature (of a surface)*: A region of a surface having a saddle shape, which curves up in one direction and curves down in a different direction, will be said to have a negative curvature. A region of a surface having a dome shape, which curves the same way in two principal directions, will be said to have a positive curvature. A flat surface will be taken to have zero curvature.

*Floppy:* A quality of a material, structure or composite that is one or more of:
- Readily conforming to finger pressure.
- Unable to retain its shape when caused to support its own weight.
- Not rigid.
- Able to be stretched or bent elastically with little effort.

The quality of being floppy may have an associated direction, hence a particular material, structure or composite may be floppy in a first direction, but stiff or rigid in a second direction, for example a second direction that is orthogonal to the first direction.

*Resilient*: Able to deform substantially elastically, and to release substantially all of the energy upon unloading, within a relatively short period of time such as 1 second.

*Rigid*: Not readily deforming to finger pressure, and/or the tensions or loads typically encountered when setting up and maintaining a patient interface in sealing relationship with an entrance to a patient's airways.

*Semi-rigid*: means being sufficiently rigid to not substantially distort under the effects of mechanical forces typically applied during respiratory pressure therapy.

### 4.5 OTHER REMARKS

A portion of the disclosure of this patent document contains material which is subject to copyright protection. The copyright owner has no objection to the facsimile reproduction by anyone of the patent document or the patent disclosure, as it appears in Patent Office patent files or records, but otherwise reserves all copyright rights whatsoever.

Unless the context clearly dictates otherwise and where a range of values is provided, it is understood that each intervening value, to the tenth of the unit of the lower limit, between the upper and lower limit of that range, and any other stated or intervening value in that stated range is encompassed within the invention. The upper and lower limits of these intervening ranges, which may be independently included in the intervening ranges, are also encompassed within the invention, subject to any specifically excluded limit in the stated range. Where the stated range includes one or both of the limits, ranges excluding either or both of those included limits are also included in the invention.

Furthermore, where a value or values are stated herein as being implemented as part of the invention, it is understood that such values may be approximated, unless otherwise stated, and such values may be utilized to any suitable significant digit to the extent that a practical technical implementation may permit or require it.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, a limited number of the exemplary methods and materials are described herein.

When a particular material is identified as being used to construct a component, obvious alternative materials with similar properties may be used as a substitute. Furthermore, unless specified to the contrary, any and all components herein described are understood to be capable of being manufactured and, as such, may be manufactured together or separately.

It must be noted that as used herein and in the appended claims, the singular forms "a", "an", and "the" include their plural equivalents, unless the context clearly dictates otherwise.

The terms "comprises" and "comprising" should be interpreted as referring to elements, components, or steps in a non-exclusive manner, indicating that the referenced elements, components, or steps may be present, or utilized, or combined with other elements, components, or steps that are not expressly referenced. Furthermore, when a relative term like "generally", "substantially", etc. is used, such a term should also include the exact term. That is, e.g. "generally" vertical should also include configurations which are exactly vertical.

The subject headings used in the detailed description are included only for the ease of reference of the reader and should not be used to limit the subject matter found throughout the disclosure or the claims. The subject headings should not be used in construing the scope of the claims or the claim limitations.

Although the invention herein has been described with reference to particular examples, it is to be understood that these examples are merely illustrative of the principles and applications of the invention. In some instances, the terminology and symbols may imply specific details that are not required to practice the invention. For example, although the terms "first" and "second" may be used, unless otherwise specified, they are not intended to indicate any order but may be utilised to distinguish between distinct elements. Furthermore, although process steps in the methodologies may be described or illustrated in an order, such an ordering is not required. Those skilled in the art will recognize that such ordering may be modified and/or aspects thereof may be conducted concurrently or even synchronously.

It is therefore to be understood that numerous modifications may be made to the illustrative examples and that other arrangements may be devised without departing from the scope of the invention.

### 4.6 REFERENCE SIGN LISTINGS

- 3000: patient interface
- 3100: seal-forming structure
- 3200: mask plenum chamber
- 3250: shell
- 3251: side wall of the shell
- 3252: lower end
- 3253: protruding web portion of the shell
- 3254: upper end
- 3256: connection portion of the shell
- 3256-1: hole of the shell
- 3256-2: groove of the shell
- 3258: locking means of the shell
- 3258-1: gear rod of the shell
- 3258-2: protrusion of the shell
- 3258-3: bendable web portion of the shell
- 3259: shoulder of the shell
- 3300: headgear
- 3500: slider of the shell
- 3800: frame
- 3810: connection portion of the frame
- 3810-1: turning protrusion of the frame
- 3810-2: sliding protrusion of the frame
- 3820: locking means of the frame
- 3820-1: protrusion of the frame
- 3820-2: gear rod of the frame
- 3830: first bar of the frame
- 3832: first portion of the first bar
- 3833: grip of the first bar
- 3834: second portion of the first bar
- 3836: third portion of the first bar
- 3836-1: portions of the third portions adjacent the shoulders
- 3837: push-area of the first bar
- 3840: second bar of the frame
- 3842: first portion of the second bar
- 3843: grip of the second bar
- 3844: second portion of the second bar
- 3846: third portion of the second bar
- 3846-1: portion of the third portion adjacent the shoulder
- 3847: push-area of the second bar
- 3850: connecting bar of the frame
- 3710: bar of the forehead support
- 3700: forehead support
- 3900: joint of the frame
- X: lateral direction in use
- Y: longitudinal direction in use
- Z: direction
- A-A: rotation axis
- A'-A': fulcrum of the curved path
- B - B: longitudinal axis of the patient interface

## Claims

1. A patient interface (3000) for delivering a supply of breathable gas to the patient's airways comprising:
a seal-forming structure (3100) adapted to seal against a user's face;
a shell (3250) being connected to the seal-forming structure (3100);
a frame (3800) adapted to be connected with a headgear (3300); wherein the seal-forming structure (3100) and the shell (3250) form at least a part of a mask plenum chamber (3200);
wherein the frame (3800) is coupled to the shell (3250) so as to allow a relative movement of the frame (3800) with respect to the shell (3250);
wherein the patient interface comprises means for providing the relative movement being configured
• as a sliding arrangement (3500) and wherein the sliding arrangement (3500) is configured such that the frame (3800) slides relative to the shell (3250) along a curved path with a fulcrum (A'-A') or
• as a joint (3900) adapted to rotate around a rotation axis (A-A); and
wherein the fulcrum (A'-A') of the sliding arrangement (3500) or the rotation axis (A-A) of the joint (3900) is located in or adjacent the center of gravity of the shell (3250) with seal-forming structure (3100).

2. The patient interface (3000) according to claim 1, further comprising a forehead support (3700) adapted to be positioned against the forehead of a patient; and wherein the frame (3800) is coupled to the forehead support (3700).

3. The patient interface (3000) according to any one of the preceding claims, further comprising means (3500, 3900) providing the relative movement, wherein the means is located between a lower end (3252) and an upper end (3254) of the shell (3250) and/or of the seal forming structure (3100).

4. The patient interface (3000) according to the preceding claim, wherein the means (3500, 3900) providing the relative movement is located approximately in the middle of the distance between the lower end (3252) and the upper end (3254) of the shell (3250) and/or of the seal forming structure (3100).

5. The patient interface (3000) according to any one of the preceding claims, wherein the frame (3800) comprises two connection portions (3810) each coupling with respective connection portions (3256) of the shell (3250) to form a pair (3256, 3810), and wherein the pairs are laterally spaced apart and wherein at least a portion of the shell (3250) and/or of the plenum chamber (3200), preferably the portion of the shell (3250) and/or of the plenum chamber (3200) receiving at least partially the nose, is/are located between the two pairs.

6. The patient interface (3000) according to any one of the preceding claims, wherein the frame (3800) comprises a first (3830) and a second (3840) bar being laterally spaced apart, wherein each bar (3830, 3840) comprises one connection portion (3810), wherein the bars (3830, 3840) each comprise a first portion (3832, 3842), and wherein the first portions (3832, 3842) extend generally parallel to the longitudinal axis (B-B) of the patient interface.

7. The patient interface (3000) according to the preceding claim, wherein the first and second bar (3830, 3840) each comprise a second portion (3834, 3844), the second portions (3834, 3844) connecting the first portions (3832, 3842) with a bar (3710) of the forehead support (3700) and wherein the second portions (3834, 3844) and the bar (3710) of the forehead support preferably form a generally Y-shaped pattern.

8. The patient interface (3000) according to any one of the preceding claims, wherein the frame (3810) comprises at least one locking means (3820) adjustably engaging with at least one locking means (3258) of the shell (3250).

9. The patient interface (3000) according to the preceding claim, wherein one of the locking means (3820, 3258) is configured as a ratchet, and wherein the other of the locking means is configured as a protrusion adapted to interengage the ratchet.

10. The patient interface (3000) according to any the preceding claim with the features of claim 6, wherein the locking means (3820) of the frame (3800) is/are configured as (a) ratchet(s) (3820-2), and wherein the locking means (3258) of the shell (3250) comprise(s) the protrusion (3258-2) engaging the ratchet(s) (3820-2)and wherein each protrusion (3258-2) is located at a bendable web portion (3258-3), the web portion (3258-3) being adapted to move the protrusion from an interlocked position to an non-interlocked position and wherein the at least one bendable web portion (3258-3) is arranged adjacent and generally parallel to the first and/or second bar(s) (3830,3840), the bendable web portion(s) being adapted to bend towards the axis and/or plane of symmetry (B-B).

11. The patient interface (3000) according to any one of the preceding claims, wherein the shell (3250) comprises at least two side walls (3251) preferably being oriented substantially parallel to each other.

12. The patient interface (3000) according to any of the preceding claims with the features of claim 3, wherein at least one connection portion (3256) of the shell (3250) and at least one connection portion (3810) of the frame (3800) form the means (3500, 3900) to allow the relative movement, wherein the connection portion (3256) of the shell (3250) is configured as a groove (3256-2) and wherein at least one connection portion (3810) of the frame (3800) is configured as a protrusion (3810-1) or a plurality of protrusions (3810-2), and wherein the shell (3250) and the frame (3800) are coupled via the at least one groove (3256-2) of the shell (3250) and the at least one protrusion (3810-2) of the frame (3800).

13. The patient interface (3000) according to any of the preceding claims, wherein, in use, the rotation axis (A-A) of the joint (3900) and/or the fulcrum (A'-A') of the sliding arrangement (3500) is/are located in a vertical direction (Y) between the sellion and the mouth of a patient, preferably at the nasal ridge, pronasale or lip superior.

## Patentansprüche

1. Patientenschnittstelle (3000) zum Abgeben einer Atemgaszufuhr zu den Atemwegen des Patienten, die aufweist:
eine Abdichtungsbildungsstruktur (3100), die geeignet ist, am Gesicht eines Benutzers abzudichten;
eine Schale (3250), die mit der Abdichtungsbildungsstruktur (3100) verbunden ist;
einen Rahmen (3800), der eingerichtet ist, mit einer Kopfmontur (3300) verbunden zu sein; wobei die Abdichtungsbildungsstruktur (3100) und die Schale (3250) mindestens einen Teil einer Masken-Luftkammer (3200) bilden;
wobei der Rahmen (3800) mit der Schale (3250) gekoppelt ist, um eine Relativbewegung des Rahmens (3800) bezüglich zur Schale (3250) zu ermöglichen;
wobei die Patientenschnittstelle eine Einrichtung zum Vorsehen der Relativbewegung aufweist, die
- als Gleitanordnung (3500) konfiguriert ist, und wobei die Gleitanordnung (3500) so konfiguriert ist, dass der Rahmen (3800) relativ zur Schale (3250) entlang eines gekrümmten Wegs mit einem Drehpunkt (A'-A') gleitet, oder
- als Gelenk (3900) konfiguriert ist, das eingerichtet ist, um eine Drehachse (A-A) zu drehen; und
wobei der Drehpunkt (A'-A') der Gleitanordnung (3500) oder die Drehachse (A-A) des Gelenks (3900) im oder benachbart zum Schwerpunkt der Schale (3250) mit Abdichtungsbildungsstruktur (3100) liegt.

2. Patientenschnittstelle (3000) nach Anspruch 1, ferner mit einer Stirnstütze (3700), die eingerichtet ist, an der Stirn eines Patienten positioniert zu sein; und wobei der Rahmen (3800) mit der Stirnstütze (3700) gekoppelt ist.

3. Patientenschnittstelle (3000) nach einem der vorstehenden Ansprüche, ferner mit einer die Relativbewegung bereitstellenden Einrichtung (3500, 3900), wobei die Einrichtung zwischen einem unteren Ende (3252) und einem oberen Ende (3254) der Schale (3250) und/oder der Abdichtungsbildungsstruktur (3100) liegt.

4. Patientenschnittstelle (3000) nach dem vorstehenden Anspruch, wobei die die Relativbewegung bereitstellende Einrichtung (3500, 3900) etwa in der Mitte des Abstands zwischen dem unteren Ende (3252) und dem oberen Ende (3254) der Schale (3250) und/oder der Abdichtungsbildungsstruktur (3100) liegt.

5. Patientenschnittstelle (3000) nach einem der vorstehenden Ansprüche, wobei der Rahmen (3800) zwei Verbindungsabschnitte (3810) aufweist, die sich jeweils mit jeweiligen Verbindungsabschnitten (3256) der Schale (3250) koppeln, um ein Paar (3256, 3810) zu bilden, und wobei die Paare seitlich beabstandet sind und wobei mindestens ein Abschnitt der Schale (3250) und/oder der Luftkammer (3200), vorzugsweise der mindestens teilweise die Nase aufnehmende Abschnitt der Schale (3250) und/oder der Luftkammer (3200), zwischen den beiden Paaren liegt.

6. Patientenschnittstelle (3000) nach einem der vorstehenden Ansprüche, wobei der Rahmen (3800) einen ersten (3830) und einen zweiten (3840) Stab aufweist, die seitlich beabstandet sind, wobei jeder Stab (3830, 3840) einen Verbindungsabschnitt (3810) aufweist, wobei die Stäbe (3830, 3840) jeweils einen ersten Abschnitt (3832, 3842) aufweisen und wobei sich die ersten Abschnitte (3832, 3842) allgemein parallel zur Längsachse (B-B) der Patientenschnittstelle erstrecken.

7. Patientenschnittstelle (3000) nach dem vorstehenden Anspruch, wobei der erste und zweite Stab (3830, 3840) jeweils einen zweiten Abschnitt (3834, 3844) aufweisen, wobei die zweiten Abschnitte (3834, 3844) die ersten Abschnitte (3832, 3842) mit einem Stab (3710) der Stirnstütze (3700) verbinden, und wobei die zweiten Abschnitte (3834, 3844) und der Stab (3710) der Stirnstütze vorzugsweise ein allgemein Y-förmiges Muster bilden.

8. Patientenschnittstelle (3000) nach einem der vorstehenden Ansprüche, wobei der Rahmen (3810) mindestens eine Verriegelungseinrichtung (3820) aufweist, die einen einstellbaren Eingriff mit mindestens einer Verriegelungseinrichtung (3258) der Schale (3250) herstellt.

9. Patientenschnittstelle (3000) nach dem vorstehenden Anspruch, wobei eine der Verriegelungseinrichtungen (3820, 3258) als Sperrstück konfiguriert ist und wobei die andere der Verriegelungseinrichtungen als Vorsprung konfiguriert ist, der geeignet ist, mit dem Sperrstück ineinander zu greifen.

10. Patientenschnittstelle (3000) nach einem der vorstehenden Ansprüche mit den Merkmalen von Anspruch 6, wobei die Verriegelungseinrichtung(en) des Rahmens (3800) als Sperrstück(e) (3820-2) konfiguriert ist (sind) und wobei die Verriegelungseinrichtung(en) (3258) der Schale (3250) den Vorsprung (3258-2) aufweist (aufweisen), der einen Eingriff mit dem (den) Sperrstück(en) (3820-2) herstellt, und wobei jeder Vorsprung (3258-2) an einem biegbaren Stegabschnitt (3258-3) liegt, wobei der Stegabschnitt (3258-3) eingerichtet ist, den Vorsprung aus einer gegenseitig verriegelten Position in eine gegenseitig entriegelte Position zu bewegen, und wobei der mindestens eine biegbare Stegabschnitt (3258-3) benachbart und allgemein parallel zum ersten und/oder zweiten Stab (3830, 3840) angeordnet ist, wobei der (die) biegbare(n) Stegabschnitt(e) eingerichtet ist (sind), sich zur Achse und/oder Symmetrieebene (B-B) zu biegen.

11. Patientenschnittstelle (3000) nach einem der vorstehenden Ansprüche, wobei die Schale (3250) mindestens zwei Seitenwände (3251) aufweist, die vorzugsweise im Wesentlichen parallel zueinander ausgerichtet sind.

12. Patientenschnittstelle (3000) nach einem der vorstehenden Ansprüche mit den Merkmalen von Anspruch 3, wobei mindestens ein Verbindungsabschnitt (3256) der Schale (3250) und mindestens ein Verbindungsabschnitt (3810) des Rahmens (3800) die Einrichtung (3500, 3900) bilden, um die Relativbewegung zu ermöglichen, wobei der Verbindungsabschnitt (3256) der Schale (3250) als Nut (3256-2) konfiguriert ist und wobei mindestens ein Verbindungsabschnitt (3810) des Rahmens (3800) als Vorsprung (3810-1) oder mehrere Vorsprünge (3810-2) konfiguriert ist und wobei die Schale (3250) und der Rahmen (3800) über die mindestens eine Nut (3256-2) der Schale (3250) und den mindestens einen Vorsprung (3810-2) des Rahmens (3800) gekoppelt sind.

13. Patientenschnittstelle (3000) nach einem der vorstehenden Ansprüche, wobei im Gebrauch die Drehachse (A-A) des Gelenks (3900) und/oder der Drehpunkt (A'-A') der Gleitanordnung (3500) in senkrechter Richtung (Y) zwischen dem Nasion und dem Mund eines Patienten liegen (liegt), vorzugsweise am Nasenrücken, der Nasenspitze oder an der Oberlippe.

## Revendications

1. Interface patient (3000) pour fournir un apport de gaz respirable aux voies respiratoires du patient, comprenant :
une structure formant un joint d'étanchéité (3100) adaptée pour assurer l'étanchéité contre le visage d'un utilisateur ;
une coque (3250) raccordée à la structure formant un joint d'étanchéité (3100) ;
un cadre (3800) adapté pour être raccordé à un casque (3300) ; dans laquelle la structure formant un joint d'étanchéité (3100) et la coque (3250) forment au moins une partie d'une chambre de répartition d'air de masque (3200) ;
dans laquelle le cadre (3800) est couplé à la coque (3250) de façon à permettre un mouvement relatif du cadre (3800) par rapport à la coque (3250) ;
dans laquelle l'interface patient comprend un moyen pour assurer le mouvement relatif qui est configuré
- en tant qu'agencement coulissant (3500) et dans laquelle l'agencement coulissant (3500) est configuré de sorte que le cadre (3800) coulisse par rapport à la coque (3250) le long d'une trajectoire courbe avec un pivot (A'-A') ou
- en tant que joint (3900) adapté pour tourner autour d'un axe de rotation (A-A) ; et dans laquelle le pivot (A'-A') de l'agencement coulissant (3500) ou l'axe de rotation (A-A) du joint (3900) est situé dans ou à proximité du centre de gravité de la coque (3250) avec la structure formant un joint d'étanchéité (3100).

2. Interface patient (3000) selon la revendication 1, comprenant en outre un support frontal (3700) adapté pour être positionné contre le front d'un patient ; et dans laquelle le cadre (3800) est couplé au support frontal (3700).

3. Interface patient (3000) selon l'une quelconque des revendications précédentes, comprenant en outre un moyen (3500, 3900) assurant le mouvement relatif, dans laquelle le moyen est situé entre une extrémité inférieure (3252) et une extrémité supérieure (3254) de la coque (3250) et/ou de la structure formant un joint d'étanchéité (3100).

4. Interface patient (3000) selon la revendication précédente, dans laquelle le moyen (3500, 3900) assurant le mouvement relatif est situé approximativement au milieu de la distance entre l'extrémité inférieure (3252) et l'extrémité supérieure (3254) de la coque (3250) et/ou de la structure formant un joint d'étanchéité (3100).

5. Interface patient (3000) selon l'une quelconque des revendications précédentes, dans laquelle le cadre (3800) comprend deux portions de raccordement (3810) s'accouplant chacune avec des portions de raccordement (3256) respectives de la coque (3250) pour former une paire (3256, 3810), et dans laquelle les paires sont espacées l'une de l'autre latéralement et dans laquelle au moins une portion de la coque (3250) et/ou de la chambre de répartition d'air (3200), de préférence la portion de la coque (3250) et/ou de la chambre de répartition d'air (3200) recevant au moins partiellement le nez, sont situées entre les deux paires.

6. Interface patient (3000) selon l'une quelconque des revendications précédentes, dans laquelle le cadre (3800) comprend une première barre (3830) et une seconde barre (3840) espacées l'une de l'autre latéralement, dans laquelle chaque barre (3830, 3840) comprend une portion de raccordement (3810), dans laquelle les barres (3830, 3840) comprennent chacune une première portion (3832, 3842), et dans laquelle les premières portions (3832, 3842) s'étendent généralement de manière parallèle à l'axe longitudinal (B-B) de l'interface patient.

7. Interface patient (3000) selon la revendication précédente, dans laquelle la première et la seconde barre (3830, 3840) comprennent chacune une seconde portion (3834, 3844), les secondes portions (3834, 3844) raccordant les premières portions (3832, 3842) avec une barre (3710) du support frontal (3700) et dans laquelle les secondes portions (3834, 3844) et la barre (3710) du support frontal forment de préférence un motif généralement en forme de Y.

8. Interface patient (3000) selon l'une quelconque des revendications précédentes, dans laquelle le cadre (3810) comprend au moins un moyen de verrouillage (3820) venant en prise de manière ajustable avec au moins un moyen de verrouillage (3258) de la coque (3250).

9. Interface patient (3000) selon la revendication précédente, dans laquelle l'un des moyens de verrouillage (3820, 3258) est configuré en tant que cliquet, et dans laquelle l'autre des moyens de verrouillage est configuré en tant que saillie adaptée pour venir mutuellement en prise avec le cliquet.

10. Interface patient (3000) selon l'une quelconque des revendications précédentes avec les caractéristiques de la revendication 6, dans laquelle les un ou plusieurs moyens de verrouillage (3820) du cadre (3800) sont configurés en tant qu'un ou plusieurs cliquets (3820-2), et dans laquelle les un ou plusieurs moyens de verrouillage (3258) de la coque (3250) comprennent la saillie (3258-2) venant en prise avec les un ou plusieurs cliquets (3820-2) et dans laquelle chaque saillie (3258-2) est située au niveau d'une portion de bande (3258-3) pliable, la portion de bande (3258-3) étant adaptée pour déplacer la saillie depuis une position enclenchée vers une position non enclenchée et dans laquelle l'au moins une portion de bande (3258-3) pliable est agencée de manière adjacente et généralement parallèle à la première et/ou seconde barre (3830, 3840), les une ou plusieurs portions de bande pliables étant adaptées pour se plier vers l'axe et/ou le plan de symétrie (B-B).

11. Interface patient (3000) selon l'une quelconque des revendications précédentes, dans laquelle la coque (3250) comprend au moins deux parois latérales (3251) de préférence orientées de manière sensiblement parallèle l'une à l'autre.

12. Interface patient (3000) selon l'une quelconque des revendications précédentes avec les caractéristiques de la revendication 3, dans laquelle au moins une portion de raccordement (3256) de la coque (3250) et au moins une portion de raccordement (3810) du cadre (3800) forment le moyen (3500, 3900) pour permettre le mouvement relatif, dans laquelle la portion de raccordement (3256) de la coque (3250) est configurée en tant que rainure (3256-2) et dans laquelle au moins une portion de raccordement (3810) du cadre (3800) est configurée en tant que saillie (3810-1) ou pluralité de saillies (3810-2), et dans laquelle la coque (3250) et le cadre (3800) sont couplés via l'au moins une rainure (3256-2) de la coque (3250) et l'au moins une saillie (3810-2) du cadre (3800).

13. Interface patient (3000) selon l'une quelconque des revendications précédentes, dans laquelle, lors de l'utilisation, l'axe de rotation (A-A) du joint (3900) et/ou le pivot (A'-A') de l'agencement coulissant (3500) sont situés dans une direction verticale (Y) entre le sellion et la bouche d'un patient, de préférence au niveau de l'arête nasale, pronasale ou de la lèvre supérieure.
